# EUROPEAN PATENT APPLICATION

(11) **EP 4 260 863 A1**
(43) Date of publication of application: **18.10.2023**
(21) Application number: 21903660.5
(22) Date of filing: 16.11.2021
(51) Int. Cl.: A61K 35/74, A61K 9/00, A61P 25/28, A23L 33/135

(54) **COMPOSITION FOR PREVENTING OR TREATING NEUROLOGICAL DISEASES OR PSYCHIATRIC DISEASES COMPRISING VESICLES DERIVED FROM SPHINGOMONAS BACTERIA**

(30) Priority: 08.12.2020 KR 20200170221; 15.11.2021 KR 20210156952
(71) Applicant: MD Healthcare Inc., Seoul 03923 (KR)
(72) Inventor: KIM, Yoon-Keun, Paju-Si, Gyeonggi-do 10908 (KR)
(74) Representative: Dehns
(86) International application number: PCT/KR2021/016743
(87) International publication number: WO 2022/124614

(57) **Abstract**

The present invention relates to a use for preventing and treating neurological diseases or psychiatric diseases, including vesicles derived from Sphingomonas bacteria. The present inventors confirmed that, when vesicles were isolated from a culture solution of the bacteria and orally administered, the vesicles were delivered into the brain, and, when the vesicles were administered to a brain disease animal model, not only was neurogenesis increased but also interneuronal integrity was increased, and also confirmed that such therapeutic effects were induced by increasing the expression of neurotrophins and receptor genes and simultaneously increasing the expression of Sirt1, Sirt5, and Sirt7 genes which suppress cellular senescence. In addition, it has been confirmed that vesicles derived from Sphingomonas bacteria suppress the secretion of inflammatory mediators from inflammatory cells caused by neurological diseases or pathogenic factors. Thus, the vesicles derived from Sphingomonas bacteria according to the present invention ameliorate symptoms caused by brain damage, such as memory ability and spatial perception ability, when administered to a brain disease mouse model, and therefore, are expected to be usefully used as a composition for preventing, alleviating or treating neurological diseases or psychiatric diseases.

## Description

### [Technical Field]

The present invention relates to a composition for preventing or treating neurological diseases or psychiatric diseases, comprising vesicles derived from *Sphingomonas* bacteria, and more particularly, to a composition for preventing, alleviating or treating neurological diseases or psychiatric diseases using vescles derived from *Sphingomonas* bacteria.

This application claims priority to and the benefit of Korean Patent Application Nos. 10-2020-0170221 and 10-2021-0156952 filed in the Korean Intellectual Property Office on December 8, 2020 and November 15, 2021, respectively, and all the contents disclosed in the specification and drawings of the applications are incorporated in this application.

### [Background Art]

As the 21st century begins, the importance of an acute infectious disease that used to be recognized as a communicable disease in the past has decreased, whereas a chronic inflammatory disease caused by an immune or metabolic disorder in the major organs of our body has changed the pattern of diseases as a major disease that reduces the quality of life and determines the human's life expectancy. In recent years, studies have focused on the fact that an intractable disease occurs because disharmony between a human and microbiome causes abnormalities in immune and metabolic functions, resulting in chronic inflammation and abnormal cell death. In particular, as an intractable disease in the aging society of the 21st century, a neurodegenerative disease including cognitive dysfunction such as Alzheimer's disease and motor dysfunction such as Parkinson's disease and Lou Gehrig's disease; a neurodevelopmental disease such as autism and an attention-deficit hyperactivity disorder; a psychiatric disease such as an anxiety disorder, depression, and schizophrenia, and the like have become major problems for national health as major diseases that determine the quality of human life.

Repetitive stress leads to damage to nerve cells (neurons), and the subsequent abnormal death of nerve cells leads to abnormalities in brain-nervous tissue structure and function. A neurological disease, such as Alzheimer's disease, Parkinson's disease, amyotropic lateral sclerosis, Huntington's disease, epilepsy, multiple sclerosis, chronic inflammatory demyelinating polyneuropathy, and diabetic neuropathy, occurs as a result of neurodegeneration or neuroinflammation of nerve cells. Further, diseases such as Kearns-Sayre syndrome (KSS), chronic progressive external ophthalmoplegia (CPEO), mitochondrial encephalomyopathy with lactic acidosis and stroke-like episodes (MELAS), myoclonic epilepsy with ragged-red fibers (MERRF), neurogenic weakness with ataxia and retinitis pigmentosa (NARP), Leigh syndrome (LS), and mitochondrial recessive ataxia syndrome are also caused by degenerative changes in nerve cells.

Recently, it has been revealed that mental disorders such as autism, mood disorder and schizophrenia are closely associated with abdominal pain. Abdominal pain is accompanied by diarrhea and constipation, and leads to irritable bowel syndrome when repeated, which is associated with gut microbial dysbiosis. In particular, it has been reported that when an intestinal bacterial imbalance occurs due to bad food, antibiotic use, and the like, harmful intestinal microorganisms cause cracks in the healthy large intestine defense membrane, causing intestinal leakage, and then toxins derived from harmful bacteria are absorbed systemically, causing or exacerbating depression.

Further, as the results of many studies on the pathogenesis of a neurological disease and a psychiatric disease have been recently published, it has been revealed that there are problems in the key pathways of these diseases at the molecular level. Discovering and recovering the key etiological mechanisms associated with the pathogenesis of a disease gives hope that many diseases can be treated at the same time. Cellular senescence occurs while cells are repeatedly exposed to various stresses, and when the immune and metabolic functions of cells are impaired during this process, the cells undergo abnormal apoptosis. That is, the key pathophysiology of a neurodegenerative disease, a neurodevelopmental disease, and a psychiatric disease occurs as a result of abnormal death of neural stem cells or an interneuronal integrity disorder.

Energy metabolism creates the energy required to carry out functions of cells and produces various materials, thereby making proteins and lipids from the endoplasmic reticulum (ER) through ATP produced by mitochondria and supplying the materials to a required region. Cells face a variety of stresses from the moment when they are formed, and biological, chemical, physical or psychological stress induces endoplasmic reticulum (ER) stress, mitochondrial dysfunction, lysosomal damage, and the like in cells, thereby causing cell death. In particular, the accumulation of mitochondrial DNA (mtDNA) mutations and the overproduction of reactive oxygen species (ROS) due to stress promote neuronal cell senescence.

Immunity is a cellular defense mechanism against biological, chemical, physical and psychological stress, and recently, it has been known that in relation to the pathogenesis of inflammatory diseases caused by abnormal immune function, danger signals resulting from intracellular oxidative stress are recognized by nucleotide-binding oligomerization domains (NLRPs), which are pattern recognition receptors present in the cytoplasm, thereby forming inflammasomes to cause an inflammatory response and abnormal cell death.

Meanwhile, it is known that the number of microorganisms that coexist in the human body reaches 100 trillion, which is about more than that of human cells, and the number of genes of microorganisms is 100-fold larger than that of humans. A microbiota or microbiome refers to a microbial community including bacteria, archaea and eukarya present in a given habitat.

Bacteria that coexist in our bodies and bacteria that exist in the surrounding environment secrete nanometer-sized vesicles to exchange information such as genes, low molecular compounds, and proteins with other cells. The mucosa forms a physical defense membrane through which particles having a size of 200 nanometers (nm) or more cannot pass, so that bacteria coexisting in the mucosa cannot pass through the mucosa, but bacteria-derived extracellular vesicles have a size of approximately 20 to 200 nanometers, and thus relatively freely pass through epithelial cells via the mucosa to be absorbed in our bodies. Locally secreted bacterial-derived vesicles are absorbed through the epithelial cells of the mucosa to induce a local inflammatory response, and vesicles that have passed through the epithelial cells are systemically absorbed to be distributed to respective organs, and regulate immune and inflammatory responses in the distributed organs. For example, vesicles derived from pathogenic gram-negative bacteria such as *Escherichia coli* locally cause an inflammatory response and cancer, and promotes a systemic inflammatory response and blood coagulation through a vascular endothelial cell inflammatory response when absorbed into blood vessels. In addition, such vesicles are absorbed into muscle cells on which insulin acts, and the like to cause insulin resistance and diabetes. In contrast, vesicles derived from beneficial bacteria may be absorbed into specific cells of respective organs to suppress the outbreak of a disease by regulating core immune functions and metabolic dysfunction.

*Sphingomonas* bacteria are obligate aerobic gram-negative bacteria that widely inhabit the natural system such as water, soil, and plant roots. While most gram-negative bacteria have lipopolysaccharide (LPS) in their outer membrane, *Sphingomonas* bacteria have sphingolipid instead of LPS in their outer membrane, thereby making them more lipid-friendly than LPS-bearing bacteria. Furthermore, *Sphingomonas* bacteria can transfer electrons through ubiquinone 10 present in the bacterial outer membrane to produce energy similar to mitochondria. *Sphingomonas* bacteria consist of bacteria of the genera *Sphingomonas, Sphingobium, Novospingobium, Sphingosinicella,* and *Sphingopyxis.*

However, there has been no report in which vesicles derived from *Sphingomonas* bacteria are applied to the treatment of a neurological disease or a psychiatric disease to date.

### [Disclosure]

### [Technical Problem]

As a result of intensive studies to solve the problems described above in the related art, the present inventors confirmed that when *Sphingomonas* bacteria are cultured, vesicles are isolated from a culture solution, and cells are treated with the vesicles, the secretion of inflammatory mediators by pathogenic factors is remarkably suppressed. Further, the present inventors confirmed that when a disease model of brain disease induced by abnormal protein production is treated with the vesicles, not only behavioral disorders associated with cognitive function, but also abnormal protein deposition are suppressed. In addition, the present inventors confirmed that in the disease model, vesicles derived from *Sphingomonas* bacteria exhibits therapeutic effect by enhancing the proliferation and differentiation of neural stem cells, and interneuronal integrity. Furthermore, the present inventors confirmed that vesicles derived from *Sphingomonas* bacteria act on nerve cells to increase the expression of a brain-derived neurotrophic factor (BDNF) which is a neurotrophic factor inducing neurogenesis and a sirtuin protein which prevents cellular senescence caused by stress, and as a result, a therapeutic effect by neurogenesis occurs, thereby completing the present invention based on this.

Thus, an object of the present invention is to provide a pharmaceutical composition for preventing or treating a neurological disease or a psychiatric disease, comprising vesicles derived from *Sphingomonas* bacteria as an active ingredient.

Another object of the present invention is to provide a food composition for preventing or alleviating a neurological disease or a psychiatric disease, comprising vesicles derived from *Sphingomonas* bacteria as an active ingredient.

Still another object of the present invention is to provide an inhalation composition for preventing or alleviating a neurological disease or a psychiatric disease, comprising vesicles derived from *Sphingomonas* bacteria as an active ingredient.

However, a technical problem to be achieved by the present invention is not limited to the aforementioned problems, and the other problems that are not mentioned may be clearly understood by a person skilled in the art from the following description.

### [Technical Solution]

To achieve the object of the present invention as described above, the present invention provide a pharmaceutical composition for preventing or treating a neurological disease or a psychiatric disease, comprising vesicles derived from *Sphingomonas* bacteria as an active ingredient.

In an exemplary embodiment of the present invention, the neurological disease may be one or more selected from the group consisting of Alzheimer's disease, Parkinson's disease, amyotropic lateral sclerosis, Huntington's disease, epilepsy, macular degeneration, glaucoma, Kearns-Sayre syndrome (KSS), chronic progressive external ophthalmoplegia (CPEO), mitochondrial encephalomyopathy with lactic acidosis and stroke-like episodes (MELAS), myoclonic epilepsy with ragged-red fibers (MERRF), neurogenic weakness with ataxia and retinitis pigmentosa (NARP), Leigh syndrome (LS), mitochondrial recessive ataxia syndrome (MIRAS), multiple sclerosis, chronic inflammatory demyelinating polyneuropathy, dysosmia, deafness, diabetic retinopathy, and diabetic neuropathy, but is not limited thereto.

In another exemplary embodiment of the present invention, the psychiatric disease may be one or more selected from the group consisting of attention deficit hyperactivity syndrome, bipolar disorder, anxiety disorder, schizophrenia, obsessive compulsive disorder, post-traumatic stress disorder, dissociative disorder, eating disorder, substance use disorder, and personality disorder, but is not limited thereto.

In still another exemplary embodiment of the present invention, the neurological disease or the psychiatric disease may be a disease mediated by a neurotrophin, but is not limited thereto.

In yet another exemplary embodiment of the present invention, the neurotrophin may be a brain-derived neurotrophic factor (BDNF) or a nerve growth factor (NGF), but is not limited thereto.

In yet another exemplary embodiment of the present invention, the vesicles derived from *Sphingomonas* bacteria may increase the expression of one or more selected from the group consisting of Sirtuin 1 (Sirt1), SitS, and Sirt7, but are not limited thereto.

In yet another exemplary embodiment of the present invention, the *Sphingomonas* bacteria may be one or more bacteria of the genus selected from the group consisting of *Sphingomonas, Sphingobium, Novospingobium, Sphingosinicella,* and *Sphingopyxis,* but are not limited thereto.

In yet another exemplary embodiment of the present invention, the *Sphingomonas* bacteria may be one or more selected from the group consisting of *Sphingomonas abaci, Sphingomonas adhaesiva, Sphingomonas aerolata, Sphingomonas aerophila, Sphingomonas aestuarii, Sphingomonas alaskensis, Sphingomonas alpina, Sphingomonas aquatilis, Sphingomonas aromaticivorans, Sphingomonas asaccharolytica, Sphingomonas astaxanthinifaciens, Sphingomonas aurantiaca, Sphingomonas azotifigens, Sphingomonas baekryungensis, Sphingomonas capsulata, Sphingomonas canadensis, Sphingomonas changbaiensis, Sphingomonas chlorophenolica, Sphingomonas chungbukensis, Sphingomonas cloacae, Sphingomonas cynarae, Sphingomonas daechungensis, Sphingomonas desiccabilis, Sphingomonas dokdonensis, Sphingomonas echinoides, Sphingomonas elodea, Sphingomonas endophytica, Sphingomonas faeni, Sphingomonas fennica, Sphingomonas flava, Sphingomonas formosensis, Sphingomonas gei, Sphingomonas gimensis, Sphingomonas ginsengisoli, Sphingomonas ginsenosidimutans, Sphingomonas glacialis, Sphingomonas guangdongensis, Sphingomonas haloaromaticamans, Sphingomonas hankookensis, Sphingomonas herbicidovorans, Sphingomonas histidinilytica, Sphingomonas indica, Sphingomonas insulae, Sphingomonas japonica, Sphingomonas jaspsi, Sphingomonas jejuensis, Sphingomonas jinjuensis, Sphingomonas kaistensis, Sphingomonas koreensis, Sphingomonas kyeonggiensis, Sphingomonas kyungheensis, Sphingomonas lacus, Sphingomonas laterariae, Sphingomonas leidyi, Sphingomonas macrogoltabidus, Sphingomonas mali, Sphingomonas melonis, Sphingomonas molluscorum, Sphingomonas morindae, Sphingomonas mucosissima, Sphingomonas naasensis, Sphingomonas natatoria, Sphingomonas oligoaromativorans, Sphingomonas oligophenolica, Sphingomonas oryziterrae, Sphingomonas panni, Sphingomonas parapaucimobilis, Sphingomonas paucimobilis, Sphingomonas phyllosphaerae, Sphingomonas pituitosa, Sphingomonas polyaromaticivorans, Sphingomonas pruni, Sphingomonas pseudosanguinis, Sphingomonas psychrolutea, Sphingomonas rosa, Sphingomonas roseiflava, Sphingomonas rubra, Sphingomonas sanguinis, Sphingomonas sanxanigenens, Sphingomonas sediminicola, Sphingomonas soli, Sphingomonas starnbergensis, Sphingomonas stygia, Sphingomonas subarctica, Sphingomonas suberifaciens, Sphingomonas subterranea, Sphingomonas taejonensis, Sphingomonas terrae, Sphingomonas true peri, Sphingomonas ursincola, Sphingomonas vulcanisoli, Sphingomonas wittichii, Sphingomonas xenophaga, Sphingomonas xinjiangensis, Sphingomonas yabuuchiae, Sphingomonas yantingensis, Sphingomonas yanoikuyae, Sphingomonas yunnanensis, and Sphingomonas zeae,* but are not limited thereto.

As still another exemplary embodiment of the present invention, the vesicles may have an average diameter of 10 to 1000 nm, but the average diameter is not limited thereto.

In yet another exemplary embodiment of the present invention, the vesicles may be isolated from a culture solution of *Sphingomonas* bacteria, but are not limited thereto.

As yet another exemplary embodiment of the present invention, the vesicles may be naturally or artificially secreted from *Sphingomonas* bacteria, but are not limited thereto.

In addition, the present invention provide a food composition for preventing or alleviating a neurological disease or a psychiatric disease, comprising vesicles derived from *Sphingomonas* bacteria as an active ingredient.

In addition, the present invention provide an inhalation composition for preventing or alleviating a neurological disease or a psychiatric disease, comprising vesicles derived from *Sphingomonas* bacteria as an active ingredient.

Further, the present invention provides a method for preventing or treating a neurological disease or a psychiatric disease, the method comprising administering a composition comprising vesicles derived from *Sphingomonas* bacteria as an active ingredient to a subject.

In addition, the present invention provides a use of a composition comprising vesicles derived from *Sphingomonas* bacteria as an active ingredient for preventing or treating a neurological disease or a psychiatric disease.

Furthermore, the present invention provides a use of vesicles derived from *Sphingomonas* bacteria for producing a drug for preventing or treating a neurological disease or a psychiatric disease.

### [Advantageous Effects]

The present inventors confirmed that when vesicles derived from gram-negative bacteria with LPS in their outer membrane were orally administered, the vesicles were not distributed in the brain, but when vesicles derived from *Sphingomonas paucimobilis,* which is a *Sphingomonas* bacterium with sphingolipid in the outer membrane, were orally administered, the vesicles were delivered to the brain. Further, the present inventors confirmed that when vesicles derived from *Sphingomonas paucimobilis* were orally administered to a brain disease mouse model, not only behavioral disorders related to cognitive function but also abnormal protein deposition were suppressed. In addition, the present inventors confirmed that in the disease model, vesicles derived from *Sphingomonas* bacteria exhibits therapeutic effect by enhancing not only the proliferation and differentiation of neural stem cells, but also interneural integrity. Furthermore, the present inventors confirmed that vesicles derived from *Sphingomonas* bacteria act on nerve cells to increase the expression of a BDNF which is a neurotrophic factor inducing neurogenesis and a sirtuin protein which prevents cellular senescence caused by stress, and as a result, a therapeutic effect by neurogenesis occurs.

Thus, it is expected that the vesicles derived from *Sphingomonas* bacteria according to the present invention can be used as agents for preventing, alleviating or treating neurological diseases or psychiatric diseases.

### [Description of Drawings]

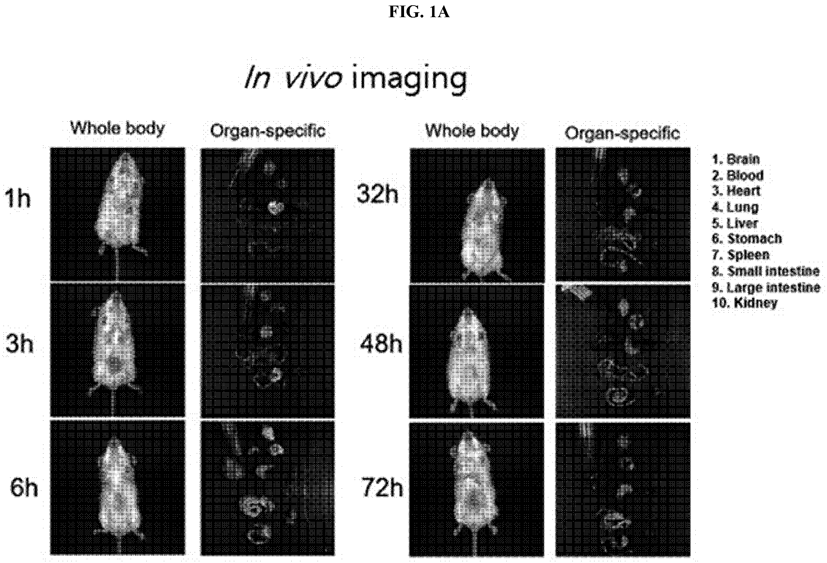

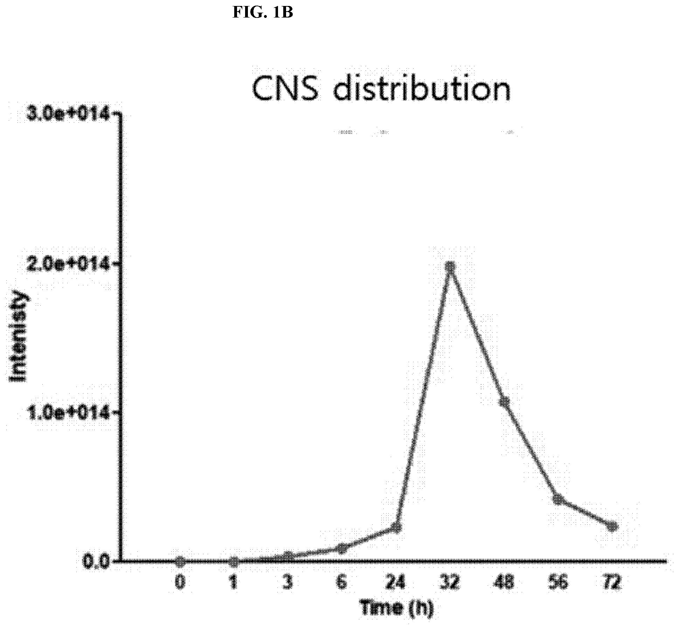

FIG. 1A is a set of photographs of the distribution pattern of vesicles derived from *Sphingomonas paucimobilis* which is a gram-negative bacterium taken over time after orally administering the vesicles to mice, and FIG. 1B shows a graph of the fluorescence intensity of vesicles derived from *Sphingomonas paucimobilis* distributed in the brain over time after oral administration.

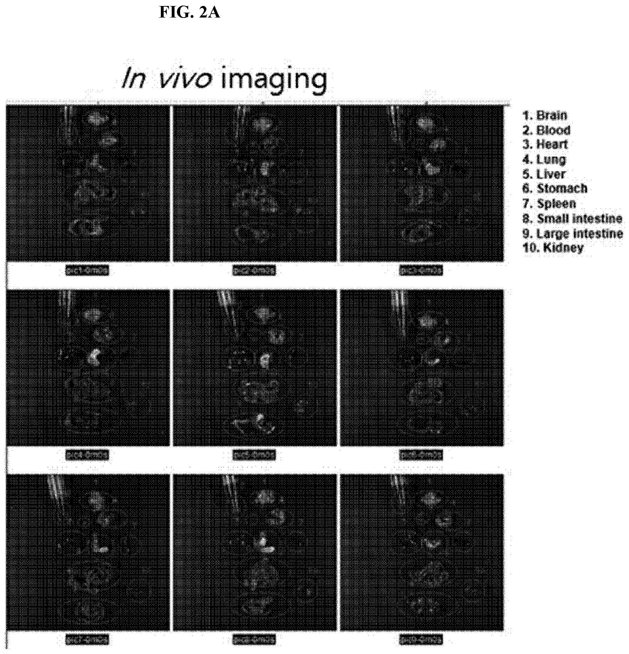

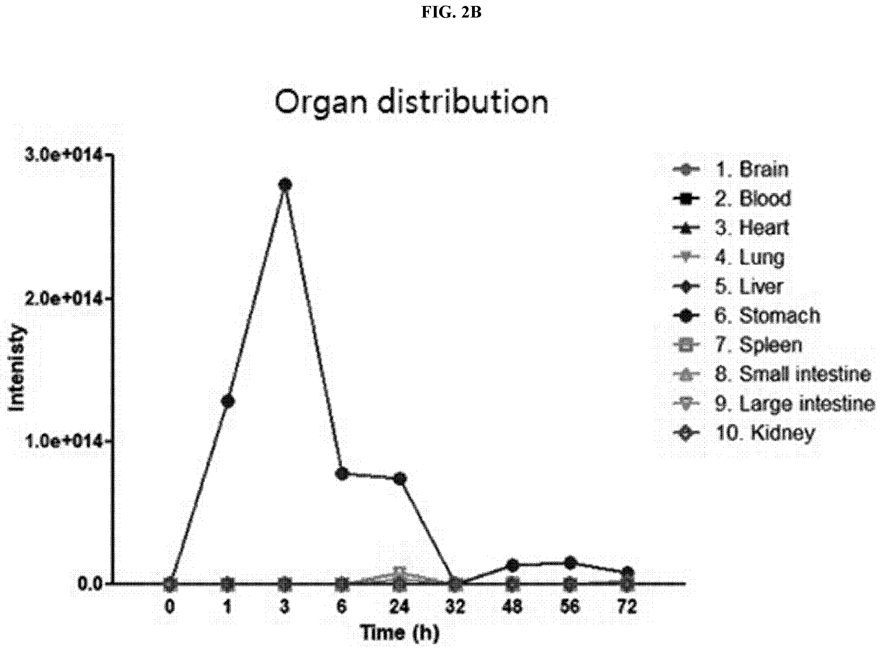

FIG. 2A is a set of photographs of the distribution pattern of vesicles derived from *Acinetobactor baumannii* which is a gram-negative bacterium taken in each organ over time after orally administering the vesicles to mice, and FIG. 2B shows a graph of the distribution pattern of vesicles derived from *Acinetobactor baumannii* in each organ over time after oral administration.

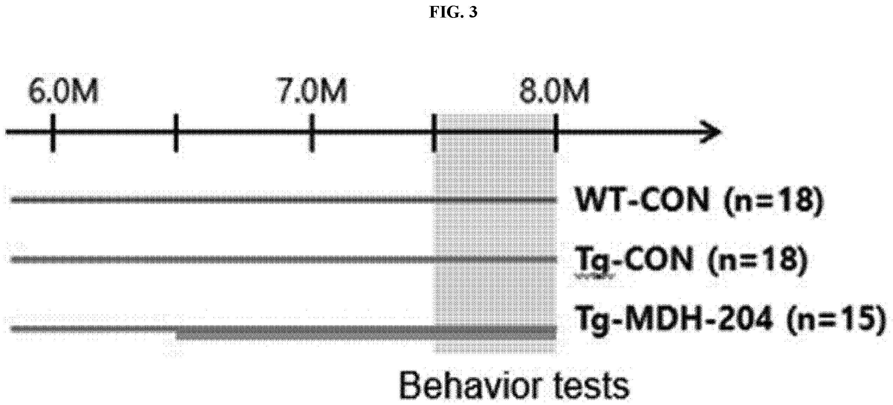

FIG. 3 is an experimental protocol for evaluating the therapeutic effect by orally administering vesicles derived from *Sphingomonas paucimobilis* in a transformed brain disease mouse model induced by overexpressing amyloid precursor protein (APP) and presenilin-1 (PSS1), which are abnormal proteins. WT-CON refers to a normal mouse group, Tg-CON refers to a brain disease mouse model group, and Tg-MDH-204 refers to a group in which vesicles derived from *Sphingomonas paucimobilis* were orally administered to a brain disease mouse model.

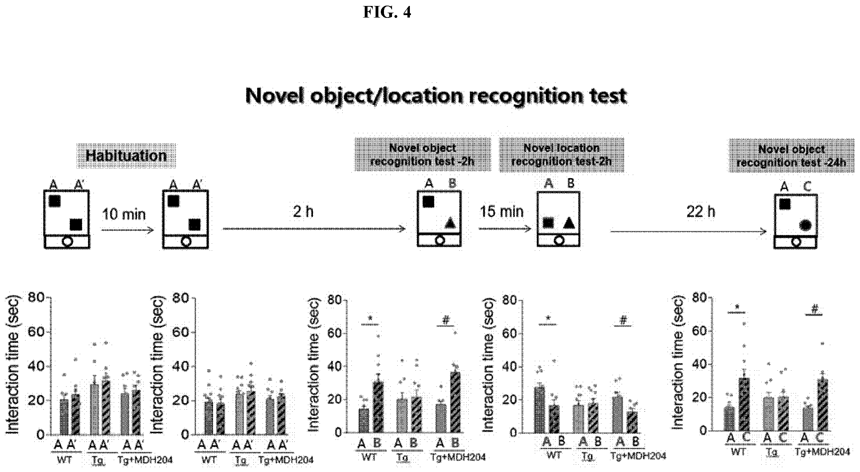

FIG. 4 shows the results of evaluating the therapeutic efficacy of *Sphingomonas paucimobilis-*derived vesicles (MDH-204) on cognitive function in an APP/PS1 transformed brain disease mouse model according to an exemplary embodiment of the present invention using a novel object/location recognition test.

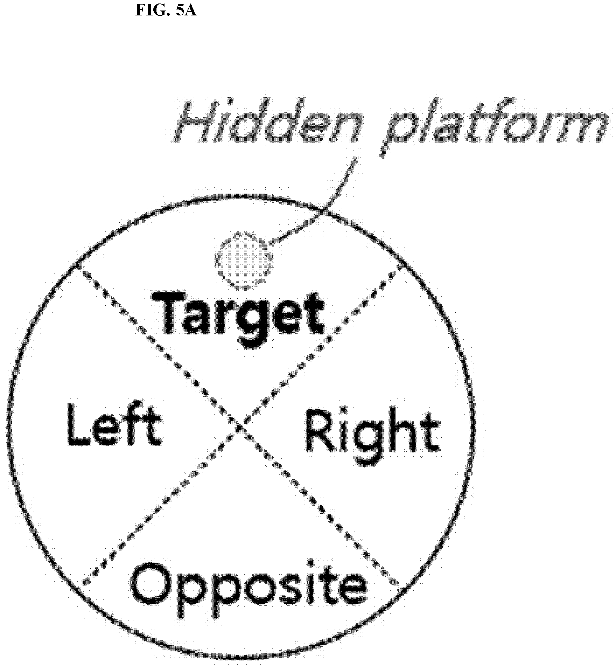

FIGS. 5A to 5C show the results of evaluating the therapeutic efficacy of *Sphingomonas paucimobilis-derived* vesicles (MDH-204) on learning ability in an APP/PS1 transformed brain disease mouse model according to an exemplary embodiment of the present invention using a water maze test.

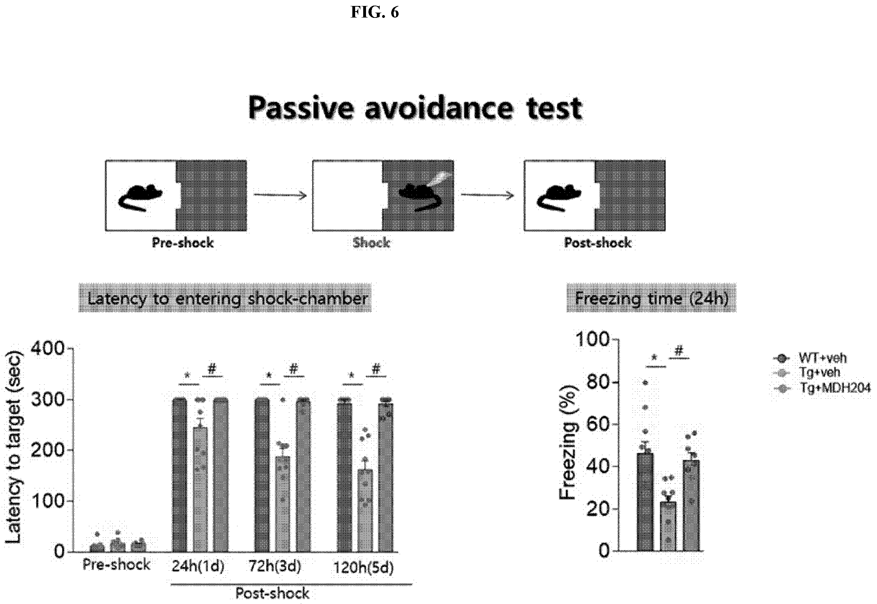

FIG. 6 shows the results of evaluating the therapeutic efficacy of *Sphingomonas paucimobilis-*derived vesicles (MDH-204) on memory ability in an APP/PS1 transformed brain disease mouse model according to an exemplary embodiment of the present invention using a passive avoidance test.

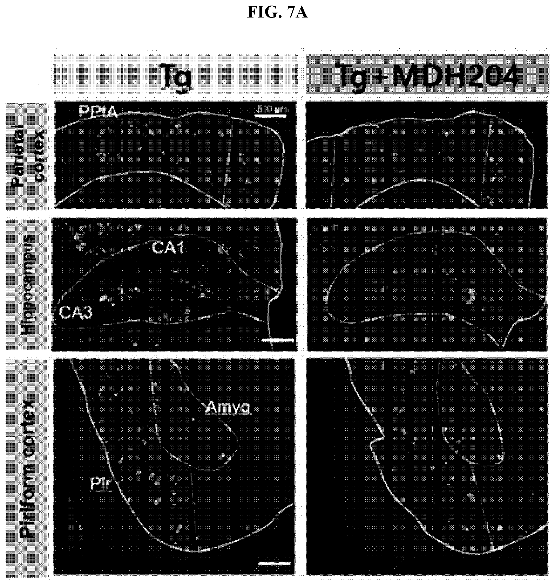

FIG. 7A to 7C show the results of evaluating the therapeutic efficacy of *Sphingomonas paucimobilis-*derived vesicles (MDH-204) on the deposition of beta amyloid, which is an abnormal protein in brain tissue in an APP/PS1 transformed brain disease mouse model according to an exemplary embodiment of the present invention.

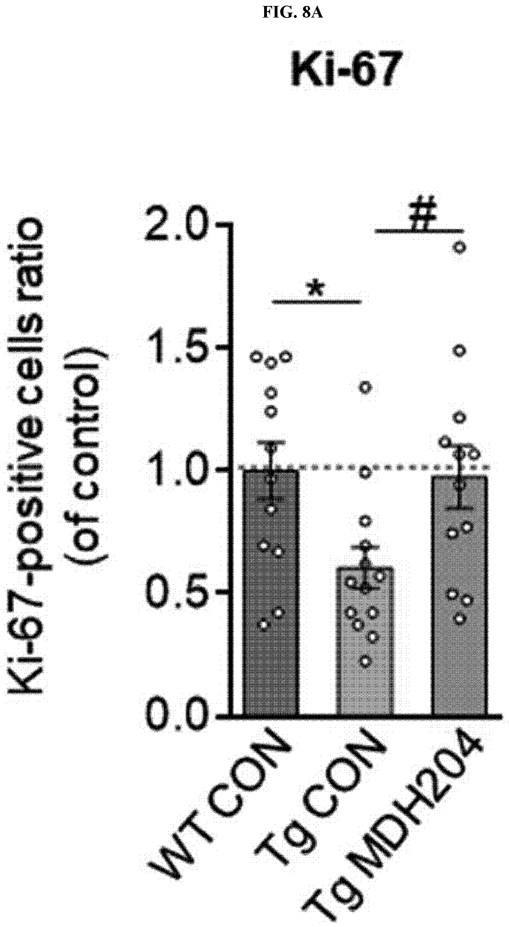

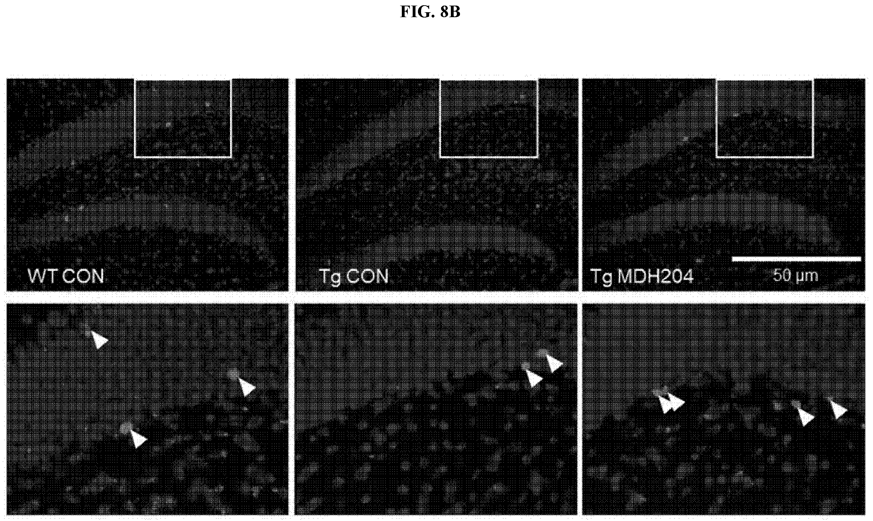

FIGS. 8A and 8B illustrate the results of showing the expression of Ki-67, which is a marker of the early neurogenesis in the brain of each group in an APP/PS1 transformed brain disease mouse model according to an exemplary embodiment of the present invention using fluorescence staining images and quantitative data, FIG. 8A illustrates the results of showing the number of cells stained with Ki-67 in a neurological disease or psychiatric disease mouse model group (Tg-CON) and a group (Tg+MDH-204) administered *Sphingomonas paucimobilis-*derived vesicles in comparison with a normal mouse group (WT-CON) as a ratio, and FIG. 8B shows a set of representative Ki-67 staining photographs for each group.

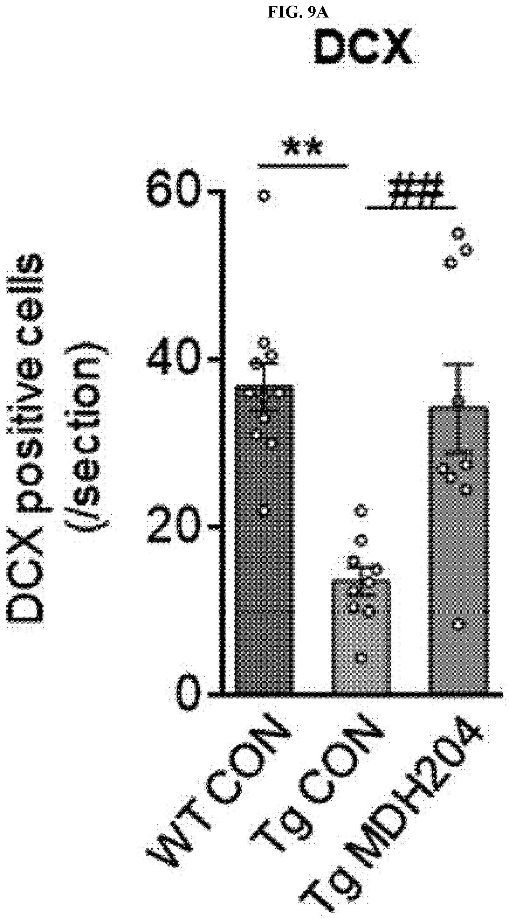

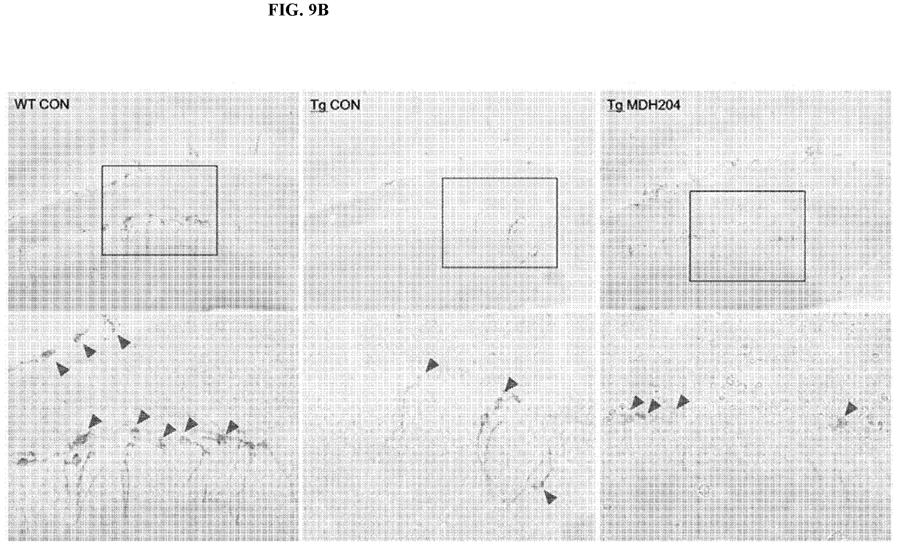

FIGS. 9A and 9B illustrate the results of showing the expression of doublecortin (DCX), which is a marker for nerve cell migration and differentiation in the brain of each group in an APP/PS1 transformed brain disease mouse model according to an exemplary embodiment of the present invention using fluorescence staining images and quantitative data, FIG. 9A illustrates the results of showing the average number of cells stained with DCX for each section observed under a microscope in a brain disease mouse model group (Tg-CON) and a group (Tg+MDH-204) administered *Sphingomonas paucimobilis-*derived vesicles in comparison with a normal mouse group (WT-CON), and FIG. 9B shows a set of representative DCX staining photographs for each group.

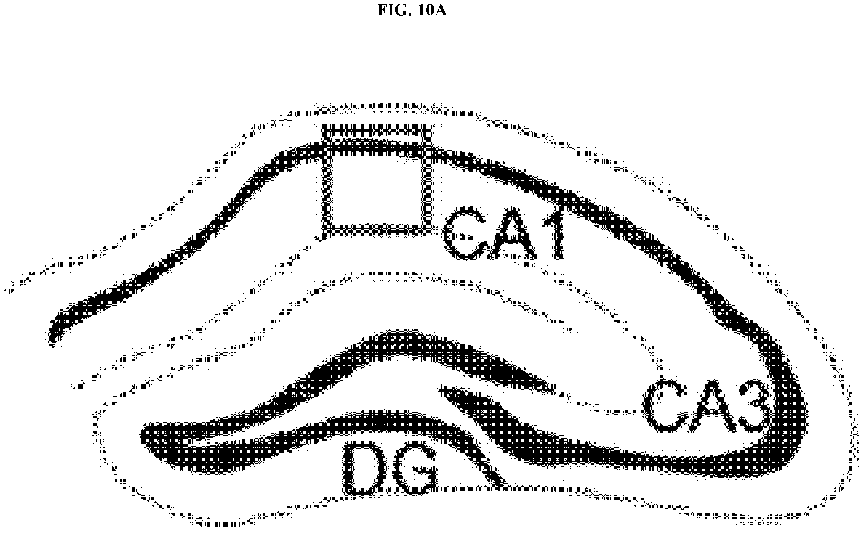

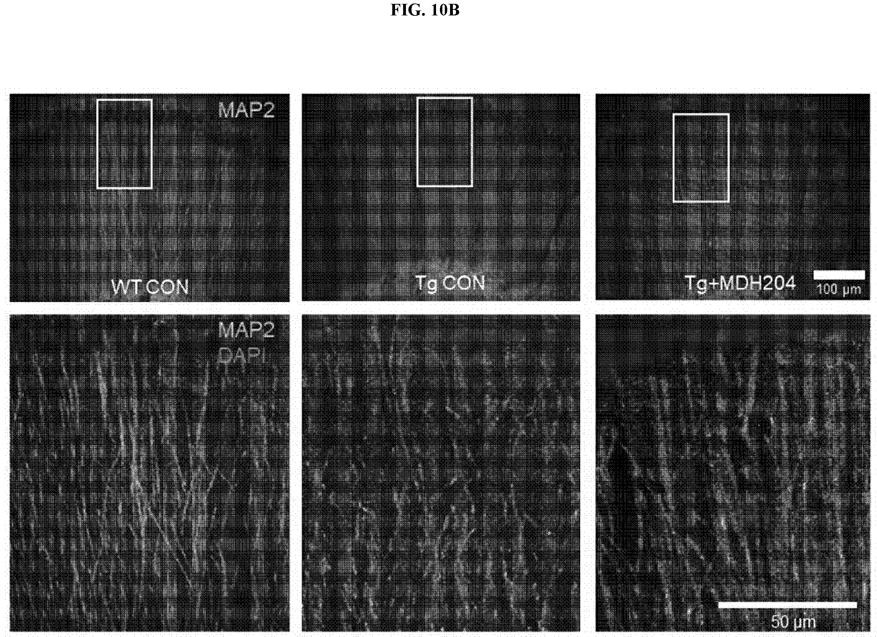

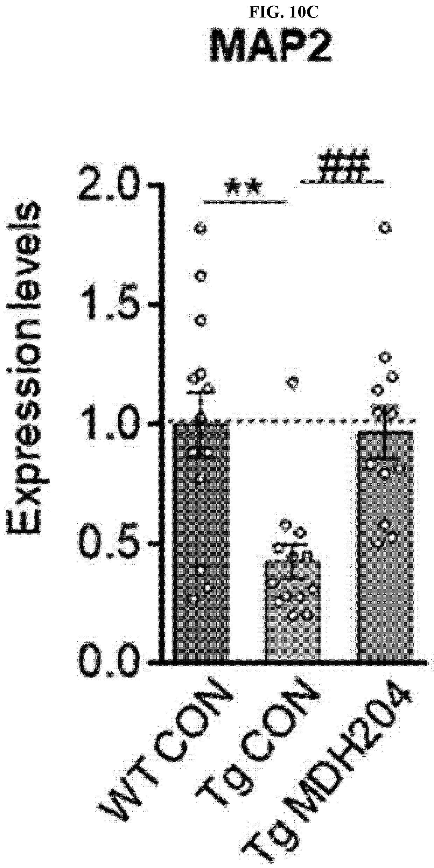

FIGS. 10A to 10C illustrates the results of showing the expression of microtubule associated protein 2 (MAP2), which is a neuronal dendrite marker, in the brain of each group in an APP/PS1 transformed brain disease mouse model according to an exemplary embodiment of the present invention using fluorescence staining images and quantitative data, FIG. 10A is a representation of the stained brain area, FIG. 10B is a set of representative MAP2 staining photographs for each group, and FIG. 10C illustrates the results of showing the expression of MAP2 in a brain disease mouse mode group (Tg-CONA) and a group (Tg+MDH-204) administered *Sphingomonas paucimobilis-*derived vesicles in comparison with a normal mouse group (WT-CON) as a ratio.

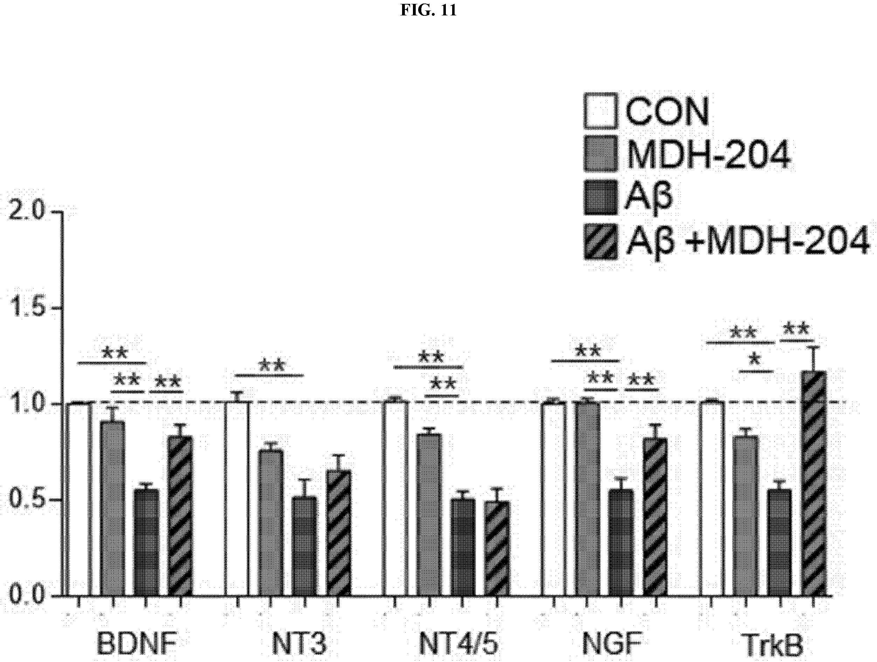

FIG. 11 illustrates the results of evaluating the therapeutic effect of *Sphingomonas paucimobilis-*derived vesicles (MDH-204) on neurogenesis and growth-related neurotrophic factors and expression of receptors by an abnormal protein beta-amyloid in nerve cells according to an exemplary embodiment of the present invention.

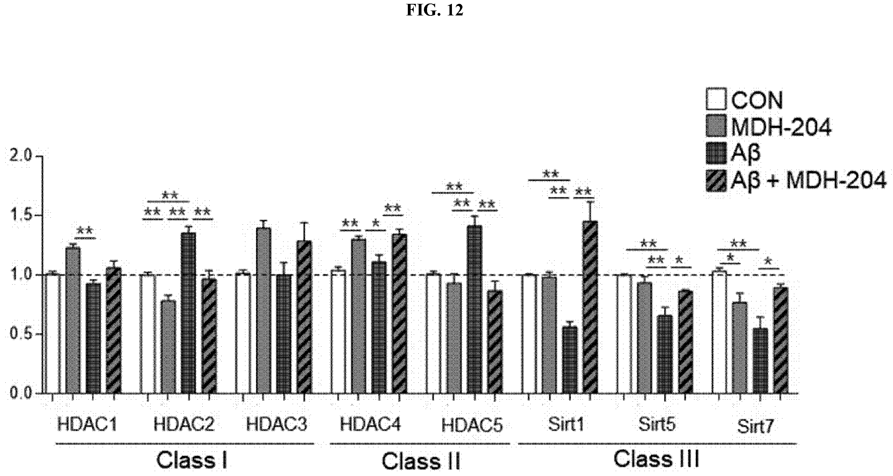

FIG. 12 illustrates the results of evaluating the therapeutic effect of *Sphingomonas paucimobilis-*derived vesicles (MDH-204) on the expression of cellular senescence-related genes by an abnormal protein beta-amyloid in nerve cells according to an exemplary embodiment of the present invention.

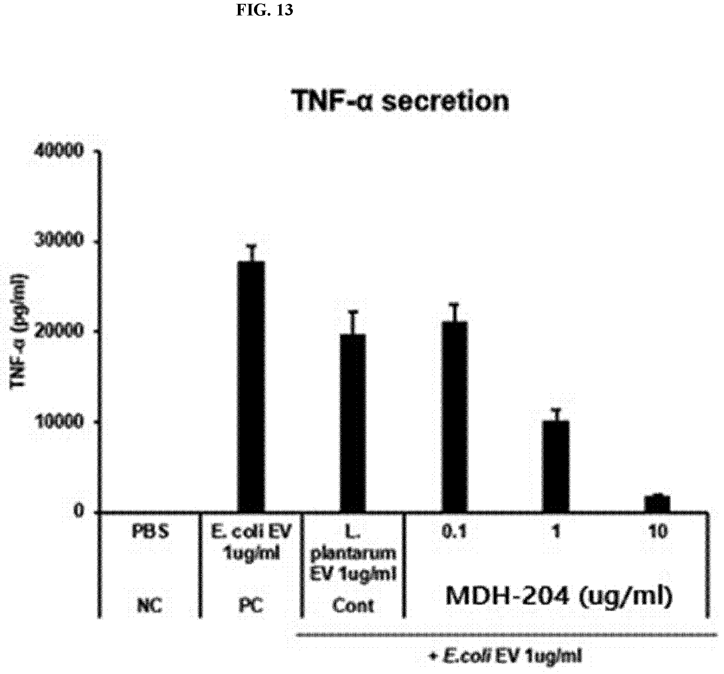

FIG. 13 illustrates the results of evaluating the therapeutic effect of *Sphingomonas paucimobilis-*derived vesicles (MDH-204) on the secretion of inflammatory mediators in inflammatory cells by pathogenic factors according to an exemplary embodiment of the present invention.

### [Best Mode]

The present invention relates to a use for preventing and treating neurological diseases or psychiatric diseases, containing vesicles derived from *Sphingomonas* bacteria.

Hereinafter, the present invention will be described in detail.

The present inventors confirmed that when vesicles derived from gram-negative bacteria with lipopolysaccharide (LPS) in their outer membrane were orally administered, the vesicles were not distributed in the brain, but when vesicles derived from *Sphingomonas* bacteria with sphingolipid in their outer membrane, were orally administered, the vesicles were delivered to the brain.

Further, the present inventors confirmed that when vesicles derived from *Sphingomonas paucimobilis* were orally administered to transgenic mice with brain disease in which abnormal proteins APP and PS1 were overexpressed, not only behavioral disorders such as cognitive function, learning ability, and memory ability but also abnormal protein deposition were suppressed.

In addition, the present inventors confirmed that in the brain disease mouse model, vesicles derived from *Sphingomonas* bacteria exhibits therapeutic effect by enhancing not only the proliferation and differentiation of neural stem cells, but also interneural integrity.

Furthermore, the present inventors confirmed that the therapeutic effects on neurogenesis and interneural integration are due to the action of vesicles derived from *Sphingomonas* bacteria on nerve cells to increase the expression of a brain-derived neurotrophic factor (BDNF), which is a neurotrophic factor, and a sirtuin protein which prevents cellular senescence.

Further, the present inventors confirmed that vesicles derived from *Sphingomonas* bacteria suppress the secretion of inflammatory mediators from inflammatory cells in a dose-dependent manner.

Therefore, the vesicles derived from *Sphingomonas* bacteria according to the present invention can be usefully used as compositions for preventing, alleviating or treating neurological diseaeses or psychiatric diseases, and the like.

Thus, the present invention provide a pharmaceutical composition for preventing, alleviating or treating a neurological disease or a psychiatric disease, comprising vesicles derived from *Sphingomonas* bacteria as an active ingredient.

The composition includes a pharmaceutical composition, a food composition, and an inhalation composition.

As used herein, the term "neurological disease or psychiatric disease" refers to a disease caused by damage and senescence of neural stem cells and nerve cells due to various stresses.

In the present invention, the neurological disease includes one or more selected from the group consisting of Alzheimer's disease, Parkinson's disease, amyotropic lateral sclerosis, Huntington's disease, epilepsy, macular degeneration, glaucoma, Kearns-Sayre syndrome (KSS), chronic progressive external ophthalmoplegia (CPEO), mitochondrial encephalomyopathy with lactic acidosis and stroke-like episodes (MELAS), myoclonic epilepsy with ragged-red fibers (MERRF), neurogenic weakness with ataxia and retinitis pigmentosa (NARP), Leigh syndrome (LS), mitochondrial recessive ataxia syndrome, multiple sclerosis, chronic inflammatory demyelinating polyneuropathy, dysosmia, deafness, diabetic retinopathy, and diabetic neuropath, but is not limited thereto.

In the present invention, the psychiatric disease refers to a pathological mental state that affects human thoughts, emotions, behaviors, and the like, and collectively refers to a state in which mental functions is impaired. The psychiatric disease includes one or more selected from the group consisting of attention deficit hyperactivity syndrome, bipolar disorder, anxiety disorder, schizophrenia, obsessive compulsive disorder, post-traumatic stress disorder, dissociative disorder, eating disorder, substance use disorder, and personality disorder, but is not limited thereto.

In the present invention, the neurological disease or the psychiatric disease may be a disease mediated by a neurotrophin, but is not limited thereto. In the present invention, the neurotrophin may be a brain-derived neurotrophic factor (BDNF) or a nerve growth factor (NGF), but is not limited thereto.

In the present invention, the vesicles derived from *Sphingomonas* bacteria may increase the expression of one or more selected from the group consisting of Sirtuin 1 (Sirt1), SitS, and Sirt7, but are not limited thereto.

In the present invention, the vesicles derived from *Sphingomonas* bacteria may have the effect of enhancing cognitive function, but are not limited thereto.

As used herein, the term "cognition" refers to all the processes of accepting and storing certain information from the brain, and searching for and using the stored information, and includes all the processes in which we think, speak, remember, judge, and practice in our lives. Cognitive function refers to the ability of the brain to remember, think, judge and practice in all processes of accepting and storing information and searching and using the stored information. Such cognitive functions may be broadly divided into attention, language, spatiotemporal, memory, and executive functions (or administrative functions). The cognitive function of the present invention includes learning ability, memory ability or concentration ability.

The "enhancement" of the present invention comprehensively refers to further improving cognitive function, which may include improving ability related to cognitive function, preferably learning ability, memory ability or concentration ability, and includes the alleviation, cure, or strengthening of symptoms such as deterioration or degeneration in cognitive function due to neurological disease or psychiatric disease, but is not limited thereto.

As used herein, the term *"Sphingomonas* bacteria" refers to obligate aerobic gram-negative bacteria that widely inhabit the natural system such as water, soil and plant roots, and includes one or more bacteria of the genus selected from the group consisting of *Sphingomonas, Sphingobium, Novospingobium, Sphingosinicella,* and *Sphingopyxis,* but is not limited thereto.

In the present invention, the *Sphingomonas* genus bacteria include one or more selected from the group consisting of *Sphingomonas abaci, Sphingomonas adhaesiva, Sphingomonas aerolata, Sphingomonas aerophila, Sphingomonas aestuarii, Sphingomonas alaskensis, Sphingomonas alpina, Sphingomonas aquatilis, Sphingomonas aromaticivorans, Sphingomonas asaccharolytica, Sphingomonas astaxanthinifaciens, Sphingomonas aurantiaca, Sphingomonas azotifigens, Sphingomonas baekryungensis, Sphingomonas capsulata, Sphingomonas canadensis, Sphingomonas changbaiensis, Sphingomonas chlorophenolica, Sphingomonas chungbukensis, Sphingomonas cloacae, Sphingomonas cynarae, Sphingomonas daechungensis, Sphingomonas desiccabilis, Sphingomonas dokdonensis, Sphingomonas echinoides, Sphingomonas elodea, Sphingomonas endophytica, Sphingomonas faeni, Sphingomonas fennica, Sphingomonas flava, Sphingomonas formosensis, Sphingomonas gei, Sphingomonas gimensis, Sphingomonas ginsengisoli, Sphingomonas ginsenosidimutans, Sphingomonas glacialis, Sphingomonas guangdongensis, Sphingomonas haloaromaticamans, Sphingomonas hankookensis, Sphingomonas herbicidovorans, Sphingomonas histidinilytica, Sphingomonas indica, Sphingomonas insulae, Sphingomonas japonica, Sphingomonas jaspsi, Sphingomonas jejuensis, Sphingomonas jinjuensis, Sphingomonas kaistensis, Sphingomonas koreensis, Sphingomonas kyeonggiensis, Sphingomonas kyungheensis, Sphingomonas lacus, Sphingomonas laterariae, Sphingomonas leidyi, Sphingomonas macrogoltabidus, Sphingomonas mali, Sphingomonas melonis, Sphingomonas molluscorum, Sphingomonas morindae, Sphingomonas mucosissima, Sphingomonas naasensis, Sphingomonas natatoria, Sphingomonas oligoaromativorans, Sphingomonas oligophenolica, Sphingomonas oryziterrae, Sphingomonas panni, Sphingomonas parapaucimobilis, Sphingomonas paucimobilis, Sphingomonas phyllosphaerae, Sphingomonas pituitosa, Sphingomonas polyaromaticivorans, Sphingomonas pruni, Sphingomonas pseudosanguinis, Sphingomonas psychrolutea, Sphingomonas rosa, Sphingomonas roseiflava, Sphingomonas rubra, Sphingomonas sanguinis, Sphingomonas sanxanigenens, Sphingomonas sediminicola, Sphingomonas soli, Sphingomonas starnbergensis, Sphingomonas stygia, Sphingomonas subarctica, Sphingomonas suberifaciens, Sphingomonas subterranea, Sphingomonas taejonensis, Sphingomonas terrae, Sphingomonas trueperi, Sphingomonas ursincola, Sphingomonas vulcanisoli, Sphingomonas wittichii, Sphingomonas xenophaga, Sphingomonas xinjiangensis, Sphingomonas yabuuchiae, Sphingomonas yantingensis, Sphingomonas yanoikuyae, Sphingomonas yunnanensis, and Sphingomonas zeae,* but are not limited thereto

As used herein, the term "extracellular vesicle" or "vesicle" refers to a structure formed of a nano-sized membrane secreted from various bacteria. Vesicles derived from gram-negative bacteria or outer membrane vesicles (OMVs) also have endotoxin (lipopolysaccharide) or glycosphingolipid, toxic proteins and bacterial DNA and RNA, and vesicles derived from gram-positive bacteria also have peptidoglycan and lipoteichoic acid which are cell wall constituents of bacteria in addition to proteins and nucleic acids. In the present invention, the vesicles may be naturally secreted from *Sphingomonas* bacteria or artificially produced by the bacteria through heat treatment, pressurization treatment, and the like, but are not limited thereto.

In the present invention, the *Sphingomonas* bacteria may include sphingolipid in the outer membrane, but are not limited thereto. Further, the vesicles derived from *Sphingomonas* bacteria may include sphingolipid, but are not limited thereto. In addition, unlike vesicles derived from other gram-negative bacteria, the vesicles derived from *Sphingomonas* bacteria may not include LPS, but are not limited thereto.

The vesicles may be isolated by heat treatment or autoclaving during *Sphingomonas* bacteria culture, or using one or more methods selected from the group consisting of centrifugation, ultracentrifugation, autoclaving, extrusion, sonication, cell lysis, homogenization, freezing-thawing, electroporation, mechanical degradation, chemical treatment, filtration with a filter, gel filtration chromatography, pre-flow electrophoresis, and capillary electrophoresis of the cell culture. In addition, for isolation, washing for removing impurities, and concentration of the obtained vesicles may be further performed.

The vesicles of the present invention may be isolated from a *Sphingomonas* bacteria culture solution or a food prepared by adding *Sphingomonas* bacteria, and the vesicles may be naturally or artificially secreted from *Sphingomonas* bacteria, but are not limited thereto.

In the present invention , the vesicles isolated by the method are in the form of spheres, and may have an average diameter of 10 to 1000 nm, 10 to 900 nm, 10 to 800 nm, 10 to 700 nm, 10 to 600 nm, 10 to 500 nm, 10 to 400 nm, 10 to 300 nm, 10 to 200 nm, 10 to 190 nm, 10 to 180 nm, 10 to 170 nm, 10 to 160 nm, 10 to 150 nm, 10 to 140 nm, 10 to 130 nm, 10 to 120 nm, 10 to 110 nm, 10 to 100 nm, 10 to 90 nm, 10 to 80 nm, 10 to 70 nm, 10 to 60 nm, 10 to 50 nm, 20 to 200 nm, 20 to 180 nm, 20 to 160 nm, 20 to 140 nm, 20 to 120 nm, 20 to 100 nm, or 20 to 80 nm, preferably 20 to 200 nm, but the average diameter is not limited thereto.

The amount of the vesicles in the composition of the present invention may be appropriately adjusted depending on the symptoms of a disease, the degree of progression of symptoms, the condition of a patient, and the like, and may range from, for example, 0.0001 wt% to 99.9 wt% or 0.001 wt% to 50 wt% with respect to a total weight of the composition, but the present invention is not limited thereto. The amount ratio is a value based on the amount of dried product from which a solvent is removed.

The pharmaceutical composition according to the present invention may further include a suitable carrier, excipient, and diluent which are commonly used in the preparation of pharmaceutical compositions. The excipient may be, for example, one or more selected from the group consisting of a diluent, a binder, a disintegrant, a lubricant, an adsorbent, a humectant, a film-coating material, and a controlled release additive.

The pharmaceutical composition according to the present invention may be used by being formulated, according to commonly used methods, into a form such as powders, granules, sustained-release-type granules, enteric granules, liquids, eye drops, elixirs, emulsions, suspensions, spirits, troches, aromatic water, lemonades, tablets, sustained-release-type tablets, enteric tablets, sublingual tablets, hard capsules, soft capsules, sustained-release-type capsules, enteric capsules, pills, tinctures, soft extracts, dry extracts, fluid extracts, injections, capsules, perfusates, or a preparation for external use, such as plasters, lotions, pastes, sprays, inhalants, patches, sterile injectable solutions, or aerosols. The preparation for external use may have a formulation such as creams, gels, patches, sprays, ointments, plasters, lotions, liniments, pastes, or cataplasmas.

As the carrier, the excipient, and the diluent that may be included in the pharmaceutical composition according to the present invention, lactose, dextrose, sucrose, oligosaccharides, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil may be used.

For formulation, commonly used diluents or excipients such as fillers, thickeners, binders, wetting agents, disintegrants, and surfactants are used.

As additives of tablets, powders, granules, capsules, pills, and troches according to the present invention, excipients such as corn starch, potato starch, wheat starch, lactose, white sugar, glucose, fructose, D-mannitol, precipitated calcium carbonate, synthetic aluminum silicate, dibasic calcium phosphate, calcium sulfate, sodium chloride, sodium hydrogen carbonate, purified lanolin, microcrystalline cellulose, dextrin, sodium alginate, methyl cellulose, sodium carboxymethylcellulose, kaolin, urea, colloidal silica gel, hydroxypropyl starch, hydroxypropyl methylcellulose (HPMC), HPMC 1928, HPMC 2208, HPMC 2906, HPMC 2910, propylene glycol, casein, calcium lactate, and *Primojel*^{®}; and binders such as gelatin, Arabic gum, ethanol, agar powder, cellulose acetate phthalate, carboxymethylcellulose, calcium carboxymethylcellulose, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, purified shellac, starch, hydroxypropyl cellulose, hydroxypropyl methylcellulose, polyvinyl alcohol, and polyvinylpyrrolidone may be used, and disintegrants such as hydroxypropyl methylcellulose, corn starch, agar powder, methylcellulose, bentonite, hydroxypropyl starch, sodium carboxymethylcellulose, sodium alginate, calcium carboxymethylcellulose, calcium citrate, sodium lauryl sulfate, silicic anhydride, 1-hydroxypropylcellulose, dextran, ion-exchange resin, polyvinyl acetate, formaldehyde-treated casein and gelatin, alginic acid, amylose, guar gum, sodium bicarbonate, polyvinylpyrrolidone, calcium phosphate, gelled starch, Arabic gum, amylopectin, pectin, sodium polyphosphate, ethyl cellulose, white sugar, magnesium aluminum silicate, a di-sorbitol solution, and light anhydrous silicic acid; and lubricants such as calcium stearate, magnesium stearate, stearic acid, hydrogenated vegetable oil, talc, lycopodium powder, kaolin, Vaseline, sodium stearate, cacao butter, sodium salicylate, magnesium salicylate, polyethylene glycol (PEG) 4000, PEG 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydride, higher fatty acids, higher alcohols, silicone oil, paraffin oil, polyethylene glycol fatty acid ether, starch, sodium chloride, sodium acetate, sodium oleate, dl-leucine, and light anhydrous silicic acid may be used.

As additives of liquids according to the present invention, water, dilute hydrochloric acid, dilute sulfuric acid, sodium citrate, monostearic acid sucrose, polyoxyethylene sorbitol fatty acid esters (twin esters), polyoxyethylene monoalkyl ethers, lanolin ethers, lanolin esters, acetic acid, hydrochloric acid, ammonia water, ammonium carbonate, potassium hydroxide, sodium hydroxide, prolamine, polyvinylpyrrolidone, ethylcellulose, and sodium carboxymethylcellulose may be used.

In syrups according to the present invention, a white sugar solution, other sugars or sweeteners, and the like may be used, and as necessary, a fragrance, a colorant, a preservative, a stabilizer, a suspending agent, an emulsifier, a viscous agent, or the like may be used.

In emulsions according to the present invention, purified water may be used, and as necessary, an emulsifier, a preservative, a stabilizer, a fragrance, or the like may be used.

In suspensions according to the present invention, suspending agents such as acacia, tragacanth, methylcellulose, carboxymethylcellulose, sodium carboxymethylcellulose, microcrystalline cellulose, sodium alginate, hydroxypropyl methylcellulose (HPMC), HPMC 1828, HPMC 2906, HPMC 2910, and the like may be used, and as necessary, a surfactant, a preservative, a stabilizer, a colorant, and a fragrance may be used.

Injections according to the present invention may include: solvents such as distilled water for injection, a 0.9% sodium chloride solution, Ringer's solution, a dextrose solution, a dextrose+sodium chloride solution, PEG, lactated Ringer's solution, ethanol, propylene glycol, non-volatile oil-sesame oil, cottonseed oil, peanut oil, soybean oil, corn oil, ethyl oleate, isopropyl myristate, and benzene benzoate; cosolvents such as sodium benzoate, sodium salicylate, sodium acetate, urea, urethane, monoethylacetamide, butazolidine, propylene glycol, the Tween series, amide nicotinate, hexamine, and dimethylacetamide; buffers such as weak acids and salts thereof (acetic acid and sodium acetate), weak bases and salts thereof (ammonia and ammonium acetate), organic compounds, proteins, albumin, peptone, and gums; isotonic agents such as sodium chloride; stabilizers such as sodium bisulfite (NaHSO₃) carbon dioxide gas, sodium metabisulfite (Na₂S₂O₅), sodium sulfite (Na₂SO₃), nitrogen gas (N₂), and ethylenediamine tetraacetic acid; sulfating agents such as 0.1% sodium bisulfide, sodium formaldehyde sulfoxylate, thiourea, disodium ethylenediaminetetraacetate, and acetone sodium bisulfite; a pain relief agent such as benzyl alcohol, chlorobutanol, procaine hydrochloride, glucose, and calcium gluconate; and suspending agents such as sodium CMC, sodium alginate, Tween 80, and aluminum monostearate.

In suppositories according to the present invention, bases such as cacao butter, lanolin, Witepsol, polyethylene glycol, glycerogelatin, methylcellulose, carboxymethylcellulose, a mixture of stearic acid and oleic acid, Subanal, cottonseed oil, peanut oil, palm oil, cacao butter + cholesterol, lecithin, lanette wax, glycerol monostearate, Tween or span, imhausen, monolan(propylene glycol monostearate), glycerin, Adeps solidus, buytyrum Tego-G, cebes Pharma 16, hexalide base 95, cotomar, Hydrokote SP, S-70-XXA, S-70-XX75(S-70-XX95), Hydrokote 25, Hydrokote 711, idropostal, massa estrarium (A, AS, B, C, D, E, I, T), masa-MF, masupol, masupol-15, neosuppostal-N, paramount-B, supposiro OSI, OSIX, A, B, C, D, H, L, suppository base IV types AB, B, A, BC, BBG, E, BGF, C, D, 299, suppostal N, Es, Wecoby W, R, S, M, Fs, and tegester triglyceride matter (TG-95, MA, 57) may be used.

Solid preparations for oral administration include tablets, pills, powders, granules, capsules, and the like, and such solid preparations are formulated by mixing the composition with at least one excipient, e.g., starch, calcium carbonate, sucrose, lactose, gelatin, and the like. In addition to simple excipients, lubricants such as magnesium stearate and talc are also used.

Examples of liquid preparations for oral administration include suspensions, liquids for internal use, emulsions, syrups, and the like, and these liquid preparations may include, in addition to simple commonly used diluents, such as water and liquid paraffin, various types of excipients, for example, a wetting agent, a sweetener, a fragrance, a preservative, and the like. Preparations for parenteral administration include an aqueous sterile solution, a non-aqueous solvent, a suspension, an emulsion, a freeze-dried preparation, and a suppository. Non-limiting examples of the non-aqueous solvent and the suspension include propylene glycol, polyethylene glycol, a vegetable oil such as olive oil, and an injectable ester such as ethyl oleate.

The pharmaceutical composition according to the present invention is administered in a pharmaceutically effective amount. In the present invention, "the pharmaceutically effective amount" refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period, and simultaneously used drugs, and factors well known in other medical fields.

The composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this may be easily determined by those of ordinary skill in the art.

The pharmaceutical composition of the present invention may be administered to a subject via various routes. All administration methods can be predicted, and the pharmaceutical composition may be administered via, for example, oral administration, subcutaneous injection, intraperitoneal injection, intravenous injection, intramuscular injection, intrathecal (space around the spinal cord) injection, sublingual administration, administration via the buccal mucosa, intrarectal insertion, intravaginal insertion, ocular administration, intra-aural administration, intranasal administration, inhalation, spraying via the mouth or nose, transdermal administration, percutaneous administration, or the like.

The pharmaceutical composition of the present invention is determined depending on the type of a drug, which is an active ingredient, along with various related factors such as a disease to be treated, administration route, the age, gender, and body weight of a patient, and the severity of diseases. Specifically, the effective amount of the composition according to the present invention may vary depending on the patient's age, sex, and body weight, and generally, 0.001 to 150 mg of the composition and preferably, 0.01 to 100 mg of the composition, per 1 kg of the body weight, may be administered daily or every other day or may be administered once to three times a day. However, since the effective amount may be increased or decreased depending on the administration route, the severity of obesity, gender, body weight, age, and the like, the dosage is not intended to limit the scope of the present invention in any way.

As used herein, the "subject" refers to a subject in need of treatment of a disease, and more specifically, refers to a mammal such as a human or a non-human primate, a mouse, a rat, a dog, a cat, a horse, and a cow, but the present invention is not limited thereto.

As used herein, the "administration" refers to providing a subject with a predetermined composition of the present invention by using an arbitrary appropriate method.

The term "prevention" as used herein means all actions that inhibit or delay the onset of a target disease. The term "treatment" as used herein means all actions that alleviate or beneficially change a target disease and abnormal metabolic symptoms caused thereby via administration of the pharmaceutical composition according to the present invention. The term "improvement" as used herein means all actions that reduce the degree of parameters related to a target disease, e.g., symptoms via administration of the composition according to the present invention.

In addition, the present invention provides a food composition for preventing or alleviating a neurological disease or a psychiatric disease, comprising vesicles derived from *Sphingomonas* bacteria as an active ingredient.

The food composition may be a health functional food composition, but is not limited thereto.

The vesicles according to the present invention may be used by adding an active ingredient as is to food or may be used together with other foods or food ingredients, but may be appropriately used according to a typical method. The mixed amount of the active ingredient may be suitably determined depending on the purpose of use thereof (for prevention or alleviation). In general, when a food or beverage is prepared, the composition of the present invention is added in an amount of 15 wt% or less, preferably 10 wt% or less based on the raw materials. However, for long-term intake for the purpose of health and hygiene or for the purpose of health control, the amount may be less than the above-mentioned range, and the vesicles have no problem in terms of stability, so the active ingredient may be used in an amount more than the above-mentioned range.

The type of food is not particularly limited. Examples of food to which the material may be added include meats, sausage, bread, chocolate, candies, snacks, confectioneries, pizza, instant noodles, other noodles, gums, dairy products including ice creams, various soups, beverages, tea, drinks, alcoholic beverages, vitamin complexes, and the like, and include all health functional foods in a typical sense.

The health beverage composition according to the present invention may contain various flavors or natural carbohydrates, and the like as additional ingredients as in a typical beverage. The above-described natural carbohydrates may be monosaccharides such as glucose and fructose, disaccharides such as maltose and sucrose, polysaccharides such as dextrin and cyclodextrin, and sugar alcohols such as xylitol, sorbitol, and erythritol. As a sweetener, it is possible to use a natural sweetener such as thaumatin and stevia extract, a synthetic sweetener such as saccharin and aspartame, and the like. The proportion of the natural carbohydrates is generally about 0.01 to 0.20 g, or about 0.04 to 0.10 g per 100 ml of the composition of the present invention.

In addition to the aforementioned ingredients, the composition of the present invention may contain various nutrients, vitamins, electrolytes, flavors, colorants, pectic acids and salts thereof, alginic acid and salts thereof, organic acids, protective colloid thickeners, pH adjusters, stabilizers, preservatives, glycerin, alcohols, carbonating agents used in carbonated drinks, and the like. In addition, the composition of the present invention may contain flesh for preparing natural fruit juice, fruit juice drinks, and vegetable drinks. These ingredients may be used either alone or in combinations thereof. The proportion of these additives is not significantly important, but is generally selected within a range of 0.01 to 0.20 part by weight per 100 parts by weight of the composition of the present invention.

Further, the present invention may be provided in the form of an inhalation composition comprising *Sphingomonas* bacteria derived vesicles as an active ingredient.

In the case of a preparation for inhalation, the compound may be formulated according to a method known in the art, and may be conveniently delivered in the form of an aerosol spray from a pressurized pack or a nebulizer by using a suitable propellant, for example, dichlorofluoromethane, trichlorofluoromethane, dichlorotetrafluoroethane, carbon dioxide, or other suitable gases. In the case of the pressurized aerosol, a dosage unit may be determined by providing a valve for transferring a metered amount. For example, a gelatin capsule and a cartridge for use in an inhaler or insufflator may be formulated so as to contain a powder mixture of a compound and a suitable powder base such as lactose or starch.

### [Modes of the Invention]

Hereinafter, preferred Examples for helping the understanding of the present invention will be suggested. However, the following Examples are provided only to more easily understand the present invention, and the contents of the present invention are not limited by the following Examples.

### [Examples]

### Example 1. Isolation of vesicles from Sphingomonas paucimobilis culture fluid

After culturing a *Sphingomonas paucimobilis* strain, vesicles thereof were isolated, analyzed and characterized. The strain was cultured in a nutrient broth (NB) medium in an incubator at 30°C until the absorbance (OD 600) became 1.0 to 1.5, and then sub-cultured in a Luria-Bertani (LB) medium. Thereafter, bacterial cells were removed by recovering a culture solution including the strain and centrifuging the culture solution at 13,000 g and 4°C for 15 minutes, and the residue was filtered with a 0.22-µm filter. The filtered supernatant was concentrated to a volume of 50 ml or less through microfiltration using a MasterFlex pump system (Cole-Parmer, US) with a 100 kDa Pellicon 2 Cassette filter membrane (Merck Millipore, US). After the concentrated supernatant was filtered with a 0.22 µm filter once again, protein was quantified using bicinchoninic acid (BCA) assay, and the following experiments were performed on the obtained vesicles (MDH-204).

### Example 2. Confirmation of pharmacokinetic characteristics of Sphingomonas paucimobilis-derived vesicles upon oral administration

In order to investigate the pharmacokinetic characteristics of *Sphingomonas paucimobilis-derived* vesicles upon oral administration, the fluorescence expressed in the body and each organ from immediately before administration to 72 hours after administration was measured by orally administering vesicles stained with a fluorescent staining reagent to mice. Fluorescence signals from the whole mouse was observed using an optical imaging system (Davinch-Invivo Fluoro Chemi (western); Davinch-K). Further, in order to evaluate the distribution pattern as the main organs after administration of the vesicles, the brain, blood, heart, lungs, liver, stomach, spleen, small intestine, large intestine and kidneys were collected to observe fluorescence signals using an optical imaging system (Davinch-Invivo Fluoro Chemi (western); Davinch-K).

As illustrated in FIG. 1A, it was confirmed that the fluorescently stained *Sphingomonas paucimobilis*-derived vesicles gradually spread in the body over time. When each organ was separately observed, a fluorescent signal of *Sphingomonas paucimobilis-*derived vesicles was observed in the stomach 1 hour after oral administration, and fluorescent signals were observed in the small intestine, large intestine, and lungs from 3 hours.

In addition, as illustrated in FIG. 1B, it was confirmed that a fluorescence signal was observed specifically in the brain from 24 hours, and this signal was strongly detected up to 32 hours and showed a tendency to gradually decrease after 48 and 72 hours passed.

Through the foregoing results, it can be seen that when *Sphingomonas paucimobilis* was orally administered, it was distributed in the central nervous system and excreted *ex vivo.*

### Example 3. Confirmation of pharmacokinetic characteristics of Acinetobactor baumannii-derived vesicles upon oral administration

In order to investigate whether the pharmacokinetic characteristics of *Sphingomonas paucimobilis-*derived vesicles are specific to bacteria with sphingolipid in their outer membrane or to gram-negative bacteria, the expressed fluorescence was measured by the same method by orally administering, to mice, *Acinetobacter baumannii-*derived vesicles, which are gram-negative bacteria-derived vesicles having LPS instead of sphingolipid in their outer membrane, stained with a fluorescent staining reagent.

As illustrated in FIG. 2A, it was confirmed that the strongest fluorescent signal was observed in the stomach 3 hours after oral administration of *Acinetobacter baumannii-*derived vesicles, and the fluorescent signal confirmed in the stomach decreased over time.

Furthermore, as illustrated in FIG. 2B, in the case of *Acinetobacter baumannii-*derived vesicles, no fluorescent signal was measured in the brain. From the above results, it can be seen that vesicles derived from gram-negative bacteria with LPS in their outer membrane and vesicles derived from gram-negative bacteria with sphingolipid in their outer membrane have very different distribution patterns in brain tissue.

### Example 4. Confirmation of cognitive function-improving effect of Sphingomonas paucimobilis-derived vesicles in mouse model of brain disease caused by abnormal protein

Amyloid precursor protein (APP) and presenilin 1 (PS1) transgenic mice are brain disease mouse models induced by overexpressing the two abnormal proteins. The brain disease mouse model is an animal model in which histologically detectable abnormal protein plaques are deposited from 6.5 months and cognitive dysfunction is detected at the time period of 7 to 8 months. Behavioral and histological examinations were performed using the mouse model after dividing the mice into a normal mouse group (WT-CON), a sham-treated brain disease mouse group (Tg-CON), and a brain disease mouse group (Tg-MDH-204) in which *Sphingomonas paucimobilis-*derived vesicles were orally administered at a dose of 50 µg/mouse as in FIG. 3.

In order to evaluate cognitive function by administration of *Sphingomonas paucimobilis-*derived vesicles in a brain disease mouse model, the time it took for the mice to find an object for 10 minutes was measured by exposing each group of WT+CON, Tg+CON, and Tg+MDH-204 to a new object or an object whose position was changed, as illustrated at the top of FIG. 4.

As illustrated in FIG. 4, it was confirmed that in a novel object recognition test (NOR) measured after 2 or 24 hours, the time to find a new object was longer in WT+CON and Tg+MDH-204, but there was no change in Tg+CON. In addition, it was confirmed that even in a novel location recognition test, it took a long time for WT+CON and Tg+MDH-204 to find the repositioned object, but there was no change in Tg+CON.

The results mean that the *Sphingomonas paucimobilis-*derived vesicles suppress the progression of short-term and long-term cognitive dysfunction in brain disease mice induced by the production of abnormal proteins.

### Example 5. Confirmation of learning ability-improving effect of Sphingomonas paucimobilis-derived vesicles in mouse model of brain disease caused by abnormal protein

In order to evaluate the learning ability efficacy by administration of *Sphingomonas paucimobilis-*derived vesicles in a mouse model of a brain disease caused by the production of abnormal proteins, as illustrated in FIG. 5A, an evaluation in which a hidden platform is found after training mice to find the hidden platform in a water bottle for 5 days was performed.

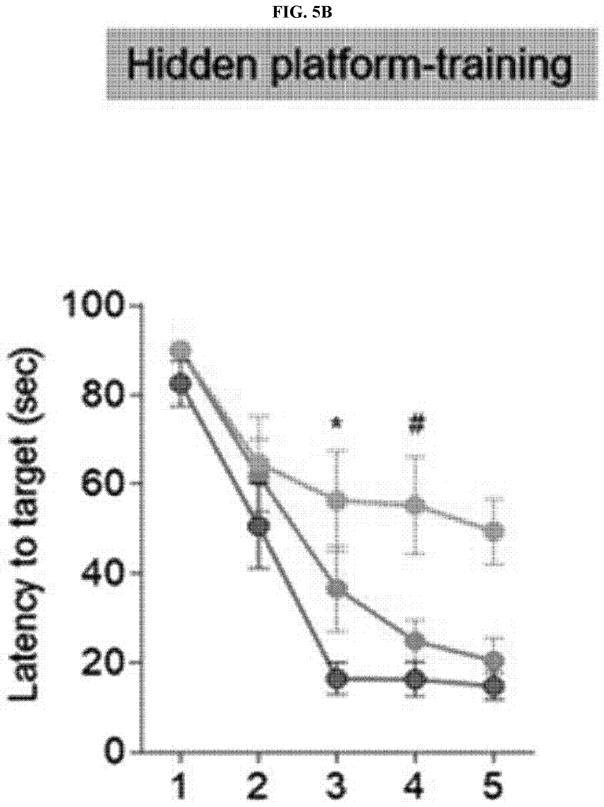

As a result, as illustrated in FIG. 5B, a normal mouse group (WT+CON, grey) had the fastest time to find the hidden platform during the training period of 5 days, a group (Tg+MDH-204, sky blue) in which *Sphingomonas paucimobilis-*derived vesicles were orally administered to a degenerative brain disease mouse model also showed a learning ability similar to that of the WT+CON group, but a sham-treated degenerative brain disease mouse model group (Tg-CON, orange) showed the slowest learning time.

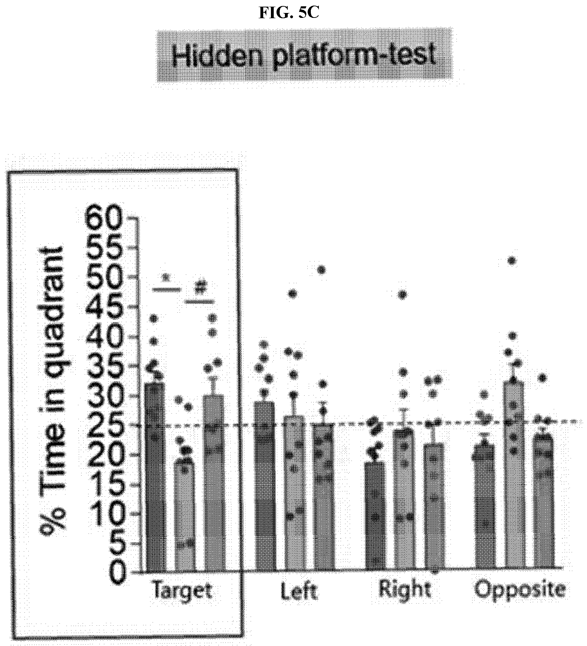

Furthermore, as illustrated in FIG. 5C, it was confirmed that even in the time to find the hidden platform and stay, the WT+CON group stayed in the platform position for a long time, and the Tg+MDH-204 group also showed to stay for a significantly longer time at the platform position than the sham-treated brain disease mouse group.

Through the results, it was confirmed that the *Sphingomonas paucimobilis-*derived vesicles have a therapeutic effect on spatial perception learning and memory ability recovery in brain disease mice.

### Example 6. Confirmation of memory ability-improving effect of Sphingomonas paucimobilis-derived vesicles in mouse model of brain disease caused by abnormal protein

In order to evaluate the efficacy on the improvement in memory ability by administration of *Sphingomonas paucimobilis-*derived vesicles in a mouse model of a brain disease caused by the production of abnormal proteins, as illustrated at the top of FIG. 6, the ability of mice to remember those associated with fear/anxiety for 24, 72, and 120 hours was evaluated after making the mice learn fear and anxiety associated with chamber context by applying an electric shock to the paws of the mice when the mice entered a dark chamber.

As a result, as illustrated in FIG. 6, it was confirmed that mice of the normal mouse group (WT+CON) and brain disease mice (Tg+MDH-204) to which *Sphingomonas paucimobilis-*derived vesicles were administered did not enter the dark chamber even after 300 seconds when the experiment passed a time point of 24, 72, and 120 hours, but the time when the sham-treated brain disease mouse group (Tg+CON) entered the dark chamber gradually became faster.

Further, when the mice entered the dark space and came out with an electric shock, the freezing time due to the shock was also significantly longer in the WT+CON group than in the sham-treated brain disease group (Tg+CON), but, there was no significant difference from the brain disease group (Tg+MDH-204) to which the vesicles were administered.

Through the results, it can be seen that the *Sphingomonas paucimobilis-*derived vesicles have the effect of suppressing memory ability loss induced by the abnormal protein.

### Example 7. Evaluation of efficacy of Sphingomonas paucimobilis-derived vesicles on formation of abnormal protein plaque in tissue in mouse model of brain disease caused by abnormal protein

Amyloid beta (Aβ) plaque is a representative abnormal protein deposited in tissues under pathological conditions, and in a Tg-APP/PS1 model, it is known that the Aβ plaque begins to accumulate in the mouse brain and induces Alzheimer's symptoms. Thus, to analyze an Aβ plaque deposited in brain tissue by administration of *Sphingomonaspaucimobilis-*derived vesicles, mouse brain sections were fluorescently stained using Thioflavin-S dye. Specifically, mice were anesthetized with 2.5% Avertin, and then perfused with 0.9% saline. Thereafter, mouse brains were fixed at 4°C using 4% paraformaldehyde (in 0.1 M phosphate buffer, pH 7.4) overnight. The fixed mouse brains were cut into 40 µm sections using Vibratom (Leica VT 1000S, Leica instruments, Mussloch, Germany). Free-floating brain sections were washed three times with 1XPBS (137 mM NaCl, 2.7 mM KCl, 10 mM Na₂HPO₄, and 1.8 mM KH₂PO₄), placed on a glass slide and dried. Thereafter, brain sections were stained with 1 mM Thioflavin S (ThS; T1892, Sigma-Aldrich) for 5 minutes. The stained slide was each washed with 100%, 95%, and 50% ethanol for 30 seconds, and then washed twice with 1X PBS. The slide was permanently mounted using a mounting medium (S3023, DAKO, Carpinteria, CA, USA), and then stored at -20°C until quantitative analysis. The stained tissue was photographed using an Olympus BX51 microscope equipped with a DP71 camera and then analyzed using MetaMorph Microscopy Automation & Image Analysis software (Molecular device) program.

As a result, as illustrated in FIG. 7A, it could be confirmed that for the formation of Aβ plaques deposited in the parietal cortex, hippocampus, and piriform cortex regions of the brain, there was a difference between the *Sphingomonas paucimobilis-*derived vesicle-treated brain disease mouse group (Tg+MDH-204) and the sham-treated brain disease mouse group (Tg+CON).

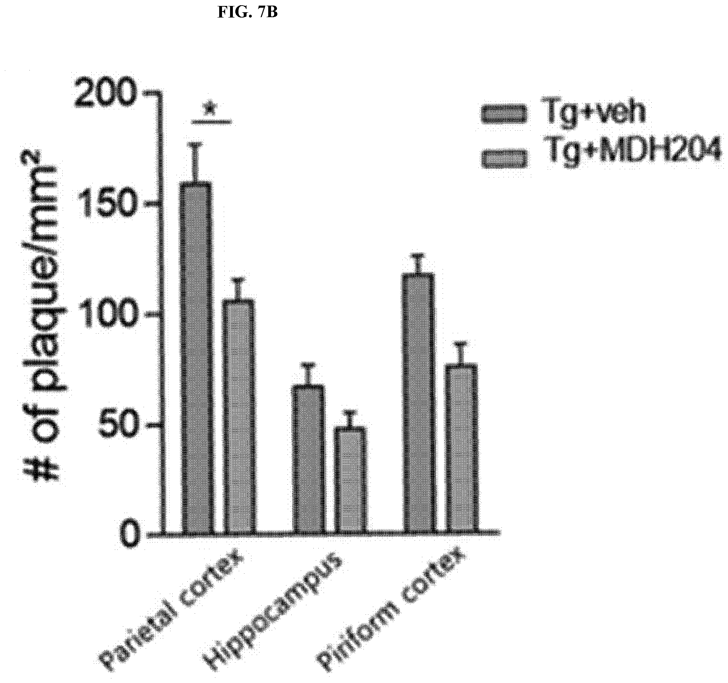

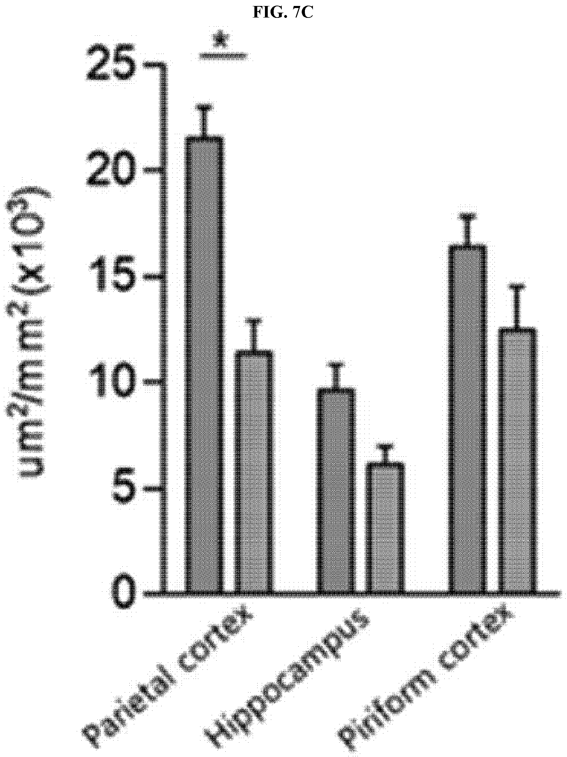

In addition, as illustrated in FIGS. 7B and 7C, it was confirmed that compared to the sham-treated brain disease mouse group (Tg+CON), the number of Aβ plaques per unit area and the Aβ plaque area, which were deposited in the parietal cortex of the brain, were significantly increased in the vesicle-treated brain disease mouse group (Tg+MDH-204).

Through the results, it can be seen that the *Sphingomonas paucimobilis-*derived vesicles have the effect of suppressing the deposition of the abnormal protein in brain tissue in the brain disease mouse model.

### Example 8. Evaluation of neurogenesis efficacy of Sphingomonas paucimobilis-derived vesicles in brain disease mouse model

Based on the above examples, in order to evaluate neurogenesis to investigate the therapeutic mechanism for the improvement of neuronal function which appeared in a brain disease mouse model administered *Sphingomonas paucimobilis-*derived vesicles, the number of cells stained with Ki67 was confirmed by fluorescently staining the cells with Ki67 known as a neural stem cell proliferation marker.

As illustrated in FIGS. 8A and 8B, a significant decrease in the number of cells stained with Ki-67 was observed in the sham-treated brain disease group (Tg+CON) compared to the normal group (WT+CON). On the other hand, the number of cells stained for Ki-67 was significantly increased in the *Sphingomonas paucimobilis-*derived vesicle-treated brain disease group (Tg+MDH-204) compared to the sham-treated brain disease group (Tg+CON), and restored to a degree similar to that of the normal mouse group.

Furthermore, in order to evaluate the efficacy of the *Sphingomonas paucimobilis-*derived vesicles in the differentiation and migration of neural stem cells, neural stem cells were subjected to fluorescence staining with double cortin (DCX), which is a marker associated with the differentiation and migration of neural stem cells.

As a result, as illustrated in FIGS. 9A and 9B, the number of DCX-positive cells was significantly reduced in the sham-administered brain disease mouse group (Tg+CON) compared to the normal mouse group (WT+CON), confirming that the number of DCX-positive cells was significantly restored in the brain disease mouse group (Tg+MDH-204) administered *Sphingomonas paucimobilis-*derived vesicles.

Through the results, it can be seen that the *Sphingomonas paucimobilis-*derived vesicles induce neurogenesis in the brain disease mouse model, and through neurogenesis, the onset and course of brain disease caused by the production of abnormal protein are suppressed.

### Example 9. Evaluation of efficacy of Sphingomonas paucimobilis-derived vesicles on interneural integrity in mouse model of brain disease caused by abnormal protein

Based on the above examples, in order to evaluate a role of interneural integrity for the improvement in nerve function shown in a brain disease mouse model to which *Sphingomonas paucimobilis-*derived vesicles were administered, the formation of dendrites (dendritic process) of differentiated nerve cells was evaluated. Since changes in the morphology and number of dendrites are important for signaling between nerve cells, the changes were evaluated using the expression of microtubule-associated protein 2 (MAP2), which is well known as a nerve cell-specific cytoskeletal protein.

As a result, as illustrated in FIGS. 10A and 10C, the expression of MAP2 was significantly reduced in the sham-administered brain disease mouse group (Tg+CON) compared to the normal mouse group (WT+CON), indicating that the expression of MAP2 was significantly restored in the group (Tg+MDH-204) in which *Sphingomonas paucimobilis-*derived vesicles were administered to brain disease mice.

Through the results, it can be seen that the *Sphingomonas paucimobilis-*derived vesicles regulate the onset or course of the brain disease by increasing the interneuronal integrity through the formation of nerve cell dendrites.

### Example 10. Confirmation of therapeutic effect of Sphingomonas paucimobilis-derived vesicles on expression of neurotrophic factor caused by abnormal protein

Upon exposure of neural stem cells and nerve cells to various stresses, the ability of neurogenesis deteriorates. Neurotrophins are a group of growth factor proteins which are essential for the proliferation of neural stem cells as well as the survival and function of differentiated nerve cells. The brains of mammals including humans produce nerve cells during the fetal period, but neural stem cells are present in the hippocampus and striatum regions, and thus, adult neurogenesis occurs even after birth. A brain-derived neurotrophic factor (BDNF), neurotrophin-3 (NT-3), NT4/5, a nerve growth factor (NGF), and the like are representative as neurotrophins associated with the survival and function of neural stem cells and nerve cells. Among them, the BDNF is a protein which is present in the central and peripheral nervous systems, and not only increases the survival and synaptic formation of nerve cells present in the brain and peripheral nervous system through TrkB receptors, but also induces the proliferation and differentiation of neural stem cells.

Thus, in order to evaluate the therapeutic mechanism of *Sphingomonas paucimobilis-*derived vesicles on neurogenesis that is suppressed by abnormal proteins, nerve cells were treated with *Sphingomonas paucimobilis-*derived vesicles, and then the expression patterns of a neurotrophin and its receptor TrkB gene were evaluated. As a method for evaluating gene expression, cells were lysed using a lysis buffer, and genes were extracted, and gene expression was quantified using RT-PCR. The degree of gene expression was evaluated using primers specific for BDNF, NT3, NT4/5, NGF, and TrkB mRNA genes. The specific sequences of the primers used for RT-PCR are shown in the following Table 1.

**[Table 1]**

| mRNA | Sequence | SEQ ID NO: |
|---|---|---|
| total Bdnf | F: TGGCTGACACTTTTGAGCAC | 1 |
| | R: GTTTGCGGCATCCAGGTAAT | 2 |
| NT3 | F: TACTACGGCAACAGAGACG | 3 |
| | R: GTTGCCCACATAATCCTCC | 4 |
| NT4/5 | F: GGCTCCATCCTGAACATCAT | 5 |
| | R: GCCATGATCTACCTGCCTGT | 6 |
| Ngf | F: AGCATTCCCTTGACACAG | 7 |
| | R: GGTCTACAGTGATGTTGC | 8 |
| TrkB | F: AAGGACTTTCATCGGGAAGCTG | 9 |
| | R: TCGCCCTCCACACAGACAC | 10 |

As a result, as illustrated in FIG. 11, when nerve cells were treated with an abnormal protein beta-amyloid (Aβ), the expression of BDNF and NGF genes was significantly increased compared to the PBS-treated negative control, and the expression of these genes was restored to the same extent as the negative control when these genes treated with the *Sphingomonas paucimobilis-*derived vesicles. Further, the expression of the BDNF receptor TrkB gene, which was suppressed by Aβ protein, was also restored by the vesicles to a degree equal to or more than that of the negative control.

Through the foregoing results, it was confirmed that *Sphingomonas paucimobilis-*derived vesicles increased neurogenesis and neuronal integrity by increasing the expression of BDNF, NGF, and their receptor TrkB genes.

### Example 11. Confirmation of therapeutic effect of Sphingomonas paucimobilis-derived vesicles on cellular senescence caused by abnormal proteins

When neural stem cells and nerve cells are repeatedly exposed to various stresses, oxidative stress causes genetic damage in the cells, leading to aging of nerve cells and abnormal death of nerve cells. Sirtuin is a signaling protein that maintains cellular homeostasis in low-calorie, stressful circumstances. Among sirtuin proteins, Sirtuin 1 is an anti-aging protein which is present in the nucleus and cytoplasm and, as a deacetylase, suppresses inflammation and increases the survival of cells upon metabolic stress. Sirtuin 5 is a protein which is present in mitochondria, has demalonylase, desuccinylase, and deacetylase enzyme functions, and regulates ammonia toxicity in mitochondria to maintain mitochondrial function. Sirtuin 7 protein is a protein which is present in the nucleolus in the nucleus, has a deacetylase enzyme function, and thus repairs rDNA damage to properly produce proteins in the ribosomes.

In addition, HDAC2 is a master regulator of genes that regulate memory, and HDAC2 is elevated in the case of neurological diseases or psychiatric diseases to block the expression of such genes, so that it is known that when HDAC2 activity is blocked or the concentration of HDAC2 is lowered, the blockage of expression of such genes required for learning and memory is prevented and the expression is restored.

Therefore, in order to evaluate the therapeutic effect of *Sphingomonas paucimobilis-*derived vesicles on the senescence of nerve cells due to oxidative stress, nerve cells were treated with *Sphingomonas paucimobilis-*derived vesicles, and then the HDAC selective suppression or the expression pattern of a sirtuin gene was evaluated. As a method for evaluating gene expression, cells were lysed using a lysis buffer, and genes were extracted, and gene expression was quantified using RT-PCR. The degree of gene expression was evaluated using the primers specific for HDAC1, HDAC2, HDAC3, HDAC4, HDAC5, Sirt1, Sirt5, and Sirt7. The specific sequences of the primers are shown in the following Table 2.

**[Table 2]**

| mRNA | Sequence | SEQ ID NO: |
|---|---|---|
| HDAC1 | F: CAGTGTGGCTCAGATTCCCT | 11 |
| | R: GGGCAGCTCATTAGGGATCT | 12 |
| HDAC2 | F: GGGACAGGCTTGGTTGTTTC | 13 |
| | R: GAGCATCAGCAATGGCAAGT | 14 |
| HDAC3 | F: AGAGAGGTCCCGAGGAGAAC | 15 |
| | R: ACTCTTGGGGACACAGCATC | 16 |
| HDAC4 | F: CAATCCCACAGTCTCCGTGT | 17 |
| | R: CAGCACCCCACTAAGGTTCA | 18 |
| HDAC5 | F: TGTCACCGCCAGATGTTTTG | 19 |
| | R: TGAGCAGAGCCGAGACACAG | 20 |
| Sirt1 | F: GATCCTTCAGTGTCATGGTTC | 21 |
| | R: ATGGCAAGTGGCTCATCA | 22 |
| Sirt5 | F: ATCGCAAGGCTGGCACCAAGAA | 23 |
| | R: CTAAAGCTGGGCAGATCGGACT | 24 |
| Sirt7 | F: GAGAGCGAGGATCTGGTGAC | 25 |
| | R: CGTGTAGACAACCAAGTGCC | 26 |

As illustrated in FIG. 12, it was confirmed that when nerve cells were treated with an abnormal protein beta-amyloid (Aβ), the expression of HDAC2 and HDAC5 genes was significantly increased compared to the PBS-treated negative control, and the expression of HDAC2 was selectively suppressed when these genes treated with the *Sphingomonas paucimobilis*-derived vesicles.

Furthermore, the expression of Sirt1, Sirt5 and Sirt7 genes was also reduced by beta-amyloid (Aβ) treatment compared to the PBS-treated negative control. On the other hand, the expression of Sirt1, Sirt5, and Sirt7 genes, whose expression was reduced by Aβ protein, was significantly increased when these genes were treated with *Sphingomonas paucimobilis*-derived vesicles.

Through the foregoing results, it was confirmed that *Sphingomonas paucimobilis-*derived vesicles not only increase the expression of Sirt1, Sirt5 and Sirt7 genes present in the nucleus, cytoplasm, and mitochondria, but also selectively suppress HDAC2 to suppress the senescence of nerve cells.

### Example 12. Confirmation of anti-inflammatory effects of Sphingomonas paucimobilis-derived vesicles

Based on the results, in order to evaluate the anti-inflammatory effects of *Sphingomonas paucimobilis-*derived vesicles, after mouse macrophage cells were pre-treated with *Sphingomonas paucimobilis-*derived vesicles at various concentrations (0.1, 1, and 10 µg/ml) for 12 hours, the cells were treated with 1 µg/ml of *E. coli*-derived vesicles, which are a pathogenic vesicle causing inflammatory diseases, and 12 hours later, the secretion of inflammatory cytokines was measured by ELISA.

For an ELISA analysis, a capture antibody was diluted in PBS, and 50 µl of the diluted capture antibody was each aliquoted into a 96-well polystyrene plate suitably for the working concentration and allowed to react at 4°C overnight. Thereafter, after the antibody was washed twice with 100 µl of PBST (PBS containing 0.05% Tween 20) solution, 100 µl of of a RD (PBST containing 1% BSA) solution was aliquoted and blocked at room temperature for 1 hour, and then after the antibody was again twice with 100 µl of PBST, 50 µl of each of the sample and the standard was aliquoted suitably for the concentration and allowed to react at room temperature for 2 hours. After the antibody was again washed twice with 100 µl of PBST, a detection antibody was diluted in RD, aliquoted in each of 50 µl suitably for the working concentration, and allowed to react at room temperature for 2 hours. After the antibody was once again washed twice with 100 µl of PBST, Strpetavidin-HRP was diluted to 1/200 in RD, aliquoted in each of 50 µl, and allowed to react at room temperature for 30 minutes. Finally, after the antibody was washed three times with 100 µl of PBST, 50 µl of a 1:1 solution of a TMB substrate and 0.04% hydrogen peroxide mixed was aliquoted, and then when color development proceeded after 5 to 20 minutes while waiting for color development, the reaction was stopped by aliquoting 50 µl of a 1 M sulfuric acid solution, and absorbance was measured at 450 nm using a Synergy^{™} HT multi-detection microplate reader (BioTek, USA).

As illustrated in FIG. 13, it was confirmed that TNF-α secretion induced by *E*. *coli*-derived vesicles was suppressed upon pre-treatment with *Sphingomonas paucimobilis-*derived vesicles. In particular, it was confirmed that the secretion suppression effect of TNF-α was dependent on the treatment concentration of *Sphingomonas paucimobilis-*derived vesicles.

This means that *Sphingomonas paucimobilis-*derived vesicles can effectively suppress inflammation-induced diseases by suppressing the secretion of inflammatory mediators secreted from inflammatory cells by pathogenic factors.

The above-described description of the present invention is provided for illustrative purposes, and those of ordinary skill in the art to which the present invention pertains will understand that the present invention can be easily modified into other specific forms without changing the technical spirit or essential features of the present invention. Therefore, it should be understood that the above-described Examples are illustrative only in all aspects and are not restrictive.

### [industrial Applicability]

The present inventors confirmed that when vesicles derived from gram-negative bacteria with LPS in their outer membrane were orally administered, the vesicles were not distributed in the brain, but when vesicles derived from *Sphingomonas paucimobilis,* which is a *Sphingomonas* bacterium with sphingolipid in the outer membrane, were orally administered, the vesicles were delivered to the brain. Further, the present inventors confirmed that when vesicles derived from *Sphingomonas paucimobilis* were orally administered to a brain disease mouse model, not only behavioral disorders related to cognitive function but also abnormal protein deposition were suppressed. In addition, the present inventors confirmed that in the disease model, vesicles derived from *Sphingomonas* bacteria exhibits therapeutic effect by enhancing not only the proliferation and differentiation of neural stem cells, but also interneural integrity. Furthermore, the present inventors confirmed that vesicles derived from *Sphingomonas* bacteria act on nerve cells to increase the expression of a BDNF which is a neurotrophic factor inducing neurogenesis and a sirtuin protein which prevents cellular senescence caused by stress, and as a result, a therapeutic effect by neurogenesis occurs. Thus, the vesicles derived from *Sphingomonas* bacteria according to the present invention can be used as agents for preventing, alleviating or treating neurological diseases or psychiatric diseases, and thus has industrial applicability.

## Claims

1. A pharmaceutical composition for preventing or treating a neurological disease or a psychiatric disease, comprising vesicles derived from *Sphingomonas* bacteria as an active ingredient.

2. The pharmaceutical composition of claim 1, wherein the neurological disease is one or more selected from the group consisting of Alzheimer's disease, Parkinson's disease, amyotropic lateral sclerosis, Huntington's disease, epilepsy, macular degeneration, glaucoma, Kearns-Sayre syndrome (KSS), chronic progressive external ophthalmoplegia (CPEO), mitochondrial encephalomyopathy with lactic acidosis and stroke-like episodes (MELAS), myoclonic epilepsy with ragged-red fibers (MERRF), neurogenic weakness with ataxia and retinitis pigmentosa (NARP), Leigh syndrome (LS), mitochondrial recessive ataxia syndrome (MIRAS), multiple sclerosis, chronic inflammatory demyelinating polyneuropathy, dysosmia, deafness, diabetic retinopathy, and diabetic neuropathy.

3. The pharmaceutical composition of claim 1, wherein the psychiatric disease is one or more selected from the group consisting of attention deficit hyperactivity syndrome, bipolar disorder, anxiety disorder, schizophrenia, obsessive compulsive disorder, post-traumatic stress disorder, dissociative disorder, eating disorder, substance use disorder, and personality disorder.

4. The pharmaceutical composition of claim 1, wherein the disease is a disease mediated by a neurotrophin.

5. The pharmaceutical composition of claim 4, wherein the neurotrophin is a brain-derived neurotrophic factor (BDNF) or a nerve growth factor (NGF).

6. The pharmaceutical composition of claim 1, wherein the vesicles derived from *Sphingomonas* bacteria is increase the expression of one or more selected from the group consisting of Sirtuin 1 (Sirt1), SitS, and Sirt7.

7. The pharmaceutical composition of claim 1, wherein the *Sphingomonas* bacteria is one or more bacteria of the genus selected from the group consisting of *Sphingomonas, Sphingobium, Novospingobium, Sphingosinicella,* and *Sphingopyxis.*

8. The pharmaceutical composition of claim 1, wherein the *Sphingomonas* bacteria is one or more selected from the group consisting of *Sphingomonas abaci, Sphingomonas adhaesiva, Sphingomonas aerolata, Sphingomonas aerophila, Sphingomonas aestuarii, Sphingomonas alaskensis, Sphingomonas alpina, Sphingomonas aquatilis, Sphingomonas aromaticivorans, Sphingomonas asaccharolytica, Sphingomonas astaxanthinifaciens, Sphingomonas aurantiaca, Sphingomonas azotifigens, Sphingomonas baekryungensis, Sphingomonas capsulata, Sphingomonas canadensis, Sphingomonas changbaiensis, Sphingomonas chlorophenolica, Sphingomonas chungbukensis, Sphingomonas cloacae, Sphingomonas cynarae, Sphingomonas daechungensis, Sphingomonas desiccabilis, Sphingomonas dokdonensis, Sphingomonas echinoides, Sphingomonas elodea, Sphingomonas endophytica, Sphingomonas faeni, Sphingomonas fennica, Sphingomonas flava, Sphingomonas formosensis, Sphingomonas gei, Sphingomonas gimensis, Sphingomonas ginsengisoli, Sphingomonas ginsenosidimutans, Sphingomonas glacialis, Sphingomonas guangdongensis, Sphingomonas haloaromaticamans, Sphingomonas hankookensis, Sphingomonas herbicidovorans, Sphingomonas histidinilytica, Sphingomonas indica, Sphingomonas insulae, Sphingomonas japonica, Sphingomonas jaspsi, Sphingomonas jejuensis, Sphingomonas jinjuensis, Sphingomonas kaistensis, Sphingomonas koreensis, Sphingomonas kyeonggiensis, Sphingomonas kyungheensis, Sphingomonas lacus, Sphingomonas laterariae, Sphingomonas leidyi, Sphingomonas macrogoltabidus, Sphingomonas mali, Sphingomonas melonis, Sphingomonas molluscorum, Sphingomonas morindae, Sphingomonas mucosissima, Sphingomonas naasensis, Sphingomonas natatoria, Sphingomonas oligoaromativorans, Sphingomonas oligophenolica, Sphingomonas oryziterrae, Sphingomonas panni, Sphingomonas parapaucimobilis, Sphingomonas paucimobilis, Sphingomonas phyllosphaerae, Sphingomonas pituitosa, Sphingomonas polyaromaticivorans, Sphingomonas pruni, Sphingomonas pseudosanguinis, Sphingomonas psychrolutea, Sphingomonas rosa, Sphingomonas roseiflava, Sphingomonas rubra, Sphingomonas sanguinis, Sphingomonas sanxanigenens, Sphingomonas sediminicola, Sphingomonas soli, Sphingomonas starnbergensis, Sphingomonas stygia, Sphingomonas subarctica, Sphingomonas suberifaciens, Sphingomonas subterranea, Sphingomonas taejonensis, Sphingomonas terrae, Sphingomonas trueperi, Sphingomonas ursincola, Sphingomonas vulcanisoli, Sphingomonas wittichii, Sphingomonas xenophaga, Sphingomonas xinjiangensis, Sphingomonas yabuuchiae, Sphingomonas yantingensis, Sphingomonas yanoikuyae, Sphingomonas yunnanensis,* and *Sphingomonas zeae.*

9. The pharmaceutical composition of claim 1, wherein the vesicles have an average diameter of 10 to 1000 nm.

10. The pharmaceutical composition of claim 1, wherein the vesicles is isolated from a culture solution of *Sphingomonas* bacteria.

11. The pharmaceutical composition of claim 1, wherein the vesicles is naturally or artificially secreted from *Sphingomonas* bacteria.

12. A food composition for preventing or alleviating a neurological disease or a psychiatric disease, comprising vesicles derived from *Sphingomonas* bacteria as an active ingredient.

13. The food composition of claim 12, wherein the neurological disease is one or more selected from the group consisting of Alzheimer's disease, Parkinson's disease, amyotropic lateral sclerosis, Huntington's disease, epilepsy, macular degeneration, glaucoma, Kearns-Sayre syndrome (KSS), chronic progressive external ophthalmoplegia (CPEO), mitochondrial encephalomyopathy with lactic acidosis and stroke-like episodes (MELAS), myoclonic epilepsy with ragged-red fibers (MERRF), neurogenic weakness with ataxia and retinitis pigmentosa (NARP), Leigh syndrome (LS), mitochondrial recessive ataxia syndrome (MIRAS), multiple sclerosis, chronic inflammatory demyelinating polyneuropathy, dysosmia, deafness, diabetic retinopathy, and diabetic neuropathy.

14. The food composition of claim 12, wherein the *Sphingomonas* bacteria is one or more bacteria of the genus selected from the group consisting of *Sphingomonas, Sphingobium, Novospingobium, Sphingosinicella,* and *Sphingopyxis.*

15. The food composition of claim 12, wherein the *Sphingomonas* bacteria is one or more selected from the group consisting of *Sphingomonas abaci, Sphingomonas adhaesiva, Sphingomonas aerolata, Sphingomonas aerophila, Sphingomonas aestuarii, Sphingomonas alaskensis, Sphingomonas alpina, Sphingomonas aquatilis, Sphingomonas aromaticivorans, Sphingomonas asaccharolytica, Sphingomonas astaxanthinifaciens, Sphingomonas aurantiaca, Sphingomonas azotifigens, Sphingomonas baekryungensis, Sphingomonas capsulata, Sphingomonas canadensis, Sphingomonas changbaiensis, Sphingomonas chlorophenolica, Sphingomonas chungbukensis, Sphingomonas cloacae, Sphingomonas cynarae, Sphingomonas daechungensis, Sphingomonas desiccabilis, Sphingomonas dokdonensis, Sphingomonas echinoides, Sphingomonas elodea, Sphingomonas endophytica, Sphingomonas faeni, Sphingomonas fennica, Sphingomonas flava, Sphingomonas formosensis, Sphingomonas gei, Sphingomonas gimensis, Sphingomonas ginsengisoli, Sphingomonas ginsenosidimutans, Sphingomonas glacialis, Sphingomonas guangdongensis, Sphingomonas haloaromaticamans, Sphingomonas hankookensis, Sphingomonas herbicidovorans, Sphingomonas histidinilytica, Sphingomonas indica, Sphingomonas insulae, Sphingomonas japonica, Sphingomonas jaspsi, Sphingomonas jejuensis, Sphingomonas jinjuensis, Sphingomonas kaistensis, Sphingomonas koreensis, Sphingomonas kyeonggiensis, Sphingomonas kyungheensis, Sphingomonas lacus, Sphingomonas laterariae, Sphingomonas leidyi, Sphingomonas macrogoltabidus, Sphingomonas mali, Sphingomonas melonis, Sphingomonas molluscorum, Sphingomonas morindae, Sphingomonas mucosissima, Sphingomonas naasensis, Sphingomonas natatoria, Sphingomonas oligoaromativorans, Sphingomonas oligophenolica, Sphingomonas oryziterrae, Sphingomonas panni, Sphingomonas parapaucimobilis, Sphingomonas paucimobilis, Sphingomonas phyllosphaerae, Sphingomonas pituitosa, Sphingomonas polyaromaticivorans, Sphingomonas pruni, Sphingomonas pseudosanguinis, Sphingomonas psychrolutea, Sphingomonas rosa, Sphingomonas roseiflava, Sphingomonas rubra, Sphingomonas sanguinis, Sphingomonas sanxanigenens, Sphingomonas sediminicola, Sphingomonas soli, Sphingomonas starnbergensis, Sphingomonas stygia, Sphingomonas subarctica, Sphingomonas suberifaciens, Sphingomonas subterranea, Sphingomonas taejonensis, Sphingomonas terrae, Sphingomonas true peri, Sphingomonas ursincola, Sphingomonas vulcanisoli, Sphingomonas wittichii, Sphingomonas xenophaga, Sphingomonas xinjiangensis, Sphingomonas yabuuchiae, Sphingomonas yantingensis, Sphingomonas yanoikuyae, Sphingomonas yunnanensis,* and *Sphingomonas zeae.*

16. The food composition of claim 12, wherein the vesicles have an average diameter of 10 to 1000 nm.

17. The food composition of claim 12, wherein the vesicles is isolated from a culture solution of *Sphingomonas* bacteria.

18. The food composition of claim 12, wherein the vesicles is naturally or artificially secreted from *Sphingomonas* bacteria.

19. An inhalation composition for preventing or alleviating a neurological disease or a psychiatric disease, comprising vesicles derived from *Sphingomonas* bacteria as an active ingredient.

20. The inhalation composition of claim 19, wherein the neurological disease is one or more selected from the group consisting of Alzheimer's disease, Parkinson's disease, amyotropic lateral sclerosis, Huntington's disease, epilepsy, macular degeneration, glaucoma, Kearns-Sayre syndrome (KSS), chronic progressive external ophthalmoplegia (CPEO), mitochondrial encephalomyopathy with lactic acidosis and stroke-like episodes (MELAS), myoclonic epilepsy with ragged-red fibers (MERRF), neurogenic weakness with ataxia and retinitis pigmentosa (NARP), Leigh syndrome (LS), mitochondrial recessive ataxia syndrome (MIRAS), multiple sclerosis, chronic inflammatory demyelinating polyneuropathy, dysosmia, deafness, diabetic retinopathy, and diabetic neuropathy.

21. The inhalation composition of claim 19, wherein the *Sphingomonas* bacteria is one or more bacteria of the genus selected from the group consisting of *Sphingomonas, Sphingobium, Novospingobium, Sphingosinicella,* and *Sphingopyxis.*

22. The inhalation composition of claim 19, wherein the *Sphingomonas* bacteria is one or more selected from the group consisting of *Sphingomonas abaci, Sphingomonas adhaesiva, Sphingomonas aerolata, Sphingomonas aerophila, Sphingomonas aestuarii, Sphingomonas alaskensis, Sphingomonas alpina, Sphingomonas aquatilis, Sphingomonas aromaticivorans, Sphingomonas asaccharolytica, Sphingomonas astaxanthinifaciens, Sphingomonas aurantiaca, Sphingomonas azotifigens, Sphingomonas baekryungensis, Sphingomonas capsulata, Sphingomonas canadensis, Sphingomonas changbaiensis, Sphingomonas chlorophenolica, Sphingomonas chungbukensis, Sphingomonas cloacae, Sphingomonas cynarae, Sphingomonas daechungensis, Sphingomonas desiccabilis, Sphingomonas dokdonensis, Sphingomonas echinoides, Sphingomonas elodea, Sphingomonas endophytica, Sphingomonas faeni, Sphingomonas fennica, Sphingomonas flava, Sphingomonas formosensis, Sphingomonas gei, Sphingomonas gimensis, Sphingomonas ginsengisoli, Sphingomonas ginsenosidimutans, Sphingomonas glacialis, Sphingomonas guangdongensis, Sphingomonas haloaromaticamans, Sphingomonas hankookensis, Sphingomonas herbicidovorans, Sphingomonas histidinilytica, Sphingomonas indica, Sphingomonas insulae, Sphingomonas japonica, Sphingomonas jaspsi, Sphingomonas jejuensis, Sphingomonas jinjuensis, Sphingomonas kaistensis, Sphingomonas koreensis, Sphingomonas kyeonggiensis, Sphingomonas kyungheensis, Sphingomonas lacus, Sphingomonas laterariae, Sphingomonas leidyi, Sphingomonas macrogoltabidus, Sphingomonas mali, Sphingomonas melonis, Sphingomonas molluscorum, Sphingomonas morindae, Sphingomonas mucosissima, Sphingomonas naasensis, Sphingomonas natatoria, Sphingomonas oligoaromativorans, Sphingomonas oligophenolica, Sphingomonas oryziterrae, Sphingomonas panni, Sphingomonas parapaucimobilis, Sphingomonas paucimobilis, Sphingomonas phyllosphaerae, Sphingomonas pituitosa, Sphingomonas polyaromaticivorans, Sphingomonas pruni, Sphingomonas pseudosanguinis, Sphingomonas psychrolutea, Sphingomonas rosa, Sphingomonas roseiflava, Sphingomonas rubra, Sphingomonas sanguinis, Sphingomonas sanxanigenens, Sphingomonas sediminicola, Sphingomonas soli, Sphingomonas starnbergensis, Sphingomonas stygia, Sphingomonas subarctica, Sphingomonas suberifaciens, Sphingomonas subterranea, Sphingomonas taejonensis, Sphingomonas terrae, Sphingomonas trueperi, Sphingomonas ursincola, Sphingomonas vulcanisoli, Sphingomonas wittichii, Sphingomonas xenophaga, Sphingomonas xinjiangensis, Sphingomonas yabuuchiae, Sphingomonas yantingensis, Sphingomonas yanoikuyae, Sphingomonas yunnanensis,* and *Sphingomonas zeae.*

23. The inhalation composition of claim 19, wherein the vesicles have an average diameter of 10 to 1000 nm.

24. The inhalation composition of claim 19, wherein the vesicles is isolated from a culture solution of *Sphingomonas* bacteria.

25. The inhalation composition of claim 19, wherein the vesicles is naturally or artificially secreted from *Sphingomonas* bacteria.

26. A method for preventing or treating a neurological disease or a psychiatric disease, the method comprising administering a composition comprising vesicles derived from *Sphingomonas* bacteria as an active ingredient to a subject.

27. A use of a composition comprising vesicles derived from *Sphingomonas* bacteria as an active ingredient for preventing or treating a neurological disease or a psychiatric disease.

28. A use of vesicles derived from *Sphingomonas* bacteria for producing a drug for preventing or treating a neurological disease or a psychiatric disease.
